(19) Europäisches Patentamt
European Patent Office
Office européen des brevets

(11) Veröffentlichungsnummer: **0 481 289 A1**

# (12) EUROPÄISCHE PATENTANMELDUNG

(21) Anmeldenummer: **91116817.7**

(22) Anmeldetag: **02.10.91**

(51) Int. Cl.$^5$: **C07C 235/88**, A01N 37/30

(30) Priorität: **15.10.90 DE 4032697**
**17.11.90 DE 4036692**

(43) Veröffentlichungstag der Anmeldung:
**22.04.92 Patentblatt 92/17**

(84) Benannte Vertragsstaaten:
**AT BE CH DE DK ES FR GB GR IT LI NL SE**

(71) Anmelder: **BASF Aktiengesellschaft**
**Carl-Bosch-Strasse 38**
**W-6700 Ludwigshafen(DE)**

(72) Erfinder: **Wagner, Oliver, Dr.**
**Kranichstrasse 9**
**W-6700 Ludwigshafen(DE)**
Erfinder: **Keil, Michael, Dr.**
**Fontanestrasse 4**
**W-6713 Freinsheim(DE)**
Erfinder: **Ammermann, Eberhard, Dr.**
**Sachsenstrasse 3**
**W-6700 Ludwigshafen(DE)**
Erfinder: **Lorenz, Gisela, Dr.**
**Erlenweg 13**
**W-6730 Neustadt(DE)**
Erfinder: **Krohn, Karsten, Prof. Dr.**
**Braunskamp 34**
**W-3300 Braunschweig(DE)**
Erfinder: **Franke, Claudia**
**Kreuzkampstrasse 1**
**W-3300 Braunschweig(DE)**

## (54) N-Fumaramidsäurederivate und diese enthaltende Fungizide.

(57) N-Fumaramidsäure-Derivate der Formel

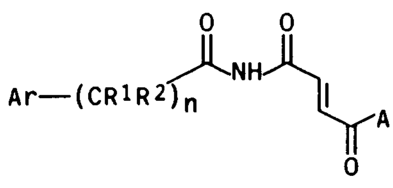

in welcher

| | |
|---|---|
| A | für O-$R^3$ oder $NR^4R^5$ steht, wobei |
| $R^3$, $R^4$, $R^5$ | Wasserstoff, Alkyl, Alkenyl, Alkinyl, Aralkyl bedeuten und der Arylrest ggf. substituiert ist, |
| $R^1$, $R^2$ | Wasserstoff, Alkyl oder $R^1$ und $R^2$ zusammen eine Methylenkette bedeuten, |
| Ar | einen Arylrest bedeutet, der ggf. substituiert ist, |
| n | für 0 - 3 steht und diese Verbindungen enthaltende Fungizide. |

EP 0 481 289 A1

Die vorliegende Erfindung betrifft neue N-(Acyl)fumaramidsäurederivate und diese enthaltende Fungizide. Aus J. Antibiotics 28, 648, (1975) und JP 51 032 789 sind bekannt:

N-(Phenylacetyl)fumarsäureamid (Ar = Ph, $R^{1,2}$ = H, A = $NH_2$)

N,N-Dimethyl-N'-(phenylacetyl)fumarsäureamid (Ar = Ph, $R^{1,2}$ = H, A = $NMe_2$)

N-([p-Methoxyphenyl]acetyl)fumarsäureamid (Ar = p-MeoPh, $R^{1,2}$ = H, A = $NH_2$)

N-([p-Nitrophenyl]acetyl)fumarsäureamid (Ar = p-$NO_2$-Ph, $R^{1,2}$ = H, A = $NH_2$)

N-(Phenylacetyl)fumaramidsäureethylester (Ar = Ph,$R^{1,2}$ = H, A = OEt).

Jedoch wird nichts über eine fungizide Wirkung berichtet.

Es wurde gefunden, daß N-Fumaramidsäurederivate der Formel 1

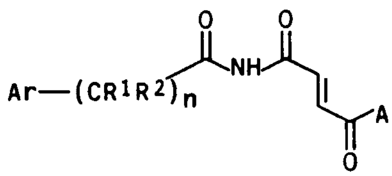

in der

| | |
|---|---|
| A | für O-$R^3$ oder $NR^4R^5$ steht, wobei |
| $R^3$, $R^4$, $R^5$ | unabhängig voneinander Wasserstoff, $C_1$-$C_6$-Alkyl, $C_1$-$C_4$-Halogenalkyl, $C_2$-$C_6$-Alkenyl, $C_2$-$C_6$-Alkinyl, Aralkyl mit 1-4 Kohlenstoffatomen im Alkylteil und der Arylrest ggf. ein- bis dreifach substituiert ist durch Substituenten aus der Gruppe $C_1$-$C_4$-Alkyl, $C_1$-$C_4$-Alkoxy, $C_1$-$C_4$-Halogenalkoxy, $C_1$-$C_4$-Halogenalkyl, Halogen, Cyan oder Nitro, bedeuten |
| $R^1$, $R^2$ | unabhängig voneinander Wasserstoff, $C_1$-$C_6$-Alkyl oder $R^1$ und $R^2$, zusammen eine Methylenkette mit 2 bis 6 Methylengruppen bedeuten, |
| Ar | einen ein- oder zweikernigen Arylrest bedeutet, der ggf. ein bis dreifach substituiert ist durch $C_1$-$C_4$-Alkyl, $C_1$-$C_4$-Alkoxy, $C_1$-$C_4$-Halogenalkyl, Phenoxy, Halogenphenoxy, Benzyloxy, Phenyl, $C_1$-$C_4$-Halogenalkoxy, Hydroxy, Halogen oder Nitro und |
| n | für 0 - 3 steht, eine gute fungizide Wirkung haben. |
| $R^1$, $R^2$ | bedeuten beispielsweise jeweils unabhängig voneinander Wasserstoff, Methyl, Ethyl, n-Propyl, Isopropyl, n-Butyl, Isobutyl, tert.-Butyl, n-Pentyl, neo-Pentyl, iso-Pentyl, n-Hexyl, |
| $R^1$, $R^2$ | können ferner zusammen mit dem C-Atom, dessen Substituenten sie sind, einen Cycloalkylring mit 3-7-C-Atomen bilden der 2-6-Methylengruppen enthält, z.B. Cyclohexyl, Cyclopropyl, |
| $R^3$, $R^4$, $R^5$ | bedeuten beispielsweise jeweils unabhängig voneinander Wasserstoff, Methyl, Ethyl, n-Propyl, Isopropyl, n-Butyl, Isobutyl, tert.-Butyl, n-Pentyl, neo-Pentyl, iso-Pentyl, n-Hexyl, Allyl, 2-Methylallyl, 3,3-Dimethylallyl, 3-Buten-1-yl, Propen-2-yl, Propargyl, Trifluormethyl, Chlormethyl, Dichlormethyl, Trichlormethyl, Brommethyl, 2-Chlorethyl, 2-Bromethyl, 3-Brompropyl, 4-Brombutyl, 4-Chlorbut-1-yl, Benzyl, 2-Fluorbenzyl, 3-Fluorbenzyl, 4-Fluorbenzyl, 2,4-Difluorbenzyl, 2-Chlorbenzyl, 3-Chlorbenzyl, 3,4-Dichlorbenzyl, 2,4-Dichlorbenzyl, 2-Chlor-4-fluorbenzyl, 2-Chlor-3-fluorbenzyl, 2-Brombenzyl, 4-Brombenzyl, 2-Brom-4-chlorbenzyl, 2-Chlor-4-methylbenzyl, 2-Trifluormethylbenzyl, 4-Trifluormethyl, 3-Trifluormethylbenzylbenzyl, 4-Methoxybenzyl, 3,4-Dimethoxybenzyl, |
| Ar | bedeutet beispielsweise Phenyl, 2-Methylphenyl, 3-Methylphenyl, 4-Methylphenyl, 2,4-Dimethylphenyl, 2,6-Dimethylphenyl, 2,4,6-Trimethylphenyl, 4-tert.-Butylphenyl, 2-Chlorphenyl, 3-Chlorphenyl, 4-Chlorphenyl, 2,4-Dichlorphenyl, 3,4-Dichlorphenyl, 2,6-Dichlorphenyl, 2,4,6-Trichlorphenyl, 2-Chlor-3-fluorphenyl, 2-Chlor-6-Fluorphenyl, 2-Chlor-4-fluorphenyl, 3-Chlor-4-methoxyphenyl, 2-Chlor-4-methylphenyl, 3-Chlor-4-Hydroxyphenyl, 2-Fluorphenyl, 3-Fluorphenyl, 4-Fluorphenyl, 2,4-Difluorphenyl, 2-Chlor-4-fluorphenyl, 3-Chlor-4-methoxyphenyl, 2-Chlor-4-methylphenyl, 3-Chlor-4-Hydroxyphenyl, 2-Fluorphenyl, 3-Fluorphenyl, 4-Fluorphenyl, 2,4-Difluorphenyl, 3-Fluor-4-methoxyphenyl, 2-Bromphenyl, 3-Bromphenyl, 4-Bromphenyl, 2-Brom-4-fluorphenyl, 2-Brom-4-chlorphenyl, 2-Methoxyphenyl, 3-Methoxyphenyl, 4-Methoxyphenyl, 4-Butoxyphenyl, 4-Propoxyphenyl, 4-tert.-Butoxyphenyl, 2-Trifluormethylphenyl, 3-Trifluormethylphenyl, 4-Trifluormethylphenyl, 4-Trifluormethoxyphenyl, 4-Tetrafluorethoxyphenyl, 3,4-Dimethoxyphenyl, 2-Methoxy-4-trifluormethylphenyl, 4-Phenoxyphenyl, 4-(4'-Chlorphenoxy)phenyl, 4-Benzyloxyphenyl, 2-Nitrophenyl, 4-Nitrophenyl, 2-Chlor-4-nitrophenyl, 4-Hydroxyphenyl, 2-Hydroxyphenyl, 1-Naphthyl, 2-Naphthyl, 4-Biphenylyl. |

n bedeutet 0 oder die ganzen Zahlen 1, 2, 3.

N-(Acyl)fumaramidsäurederivate der Formel 1 sind teilweise bekannt (J. Antibiotics 28, 648 (1975). Die dort angegebenen Versuchsvorschriften können auf die Synthese der neuen N-(Acyl)-fumaramidsäure-Derivate übertragen werden. N-(Acyl)fumaramidsäurederivate der Formel 1 mit A = O-$R_3$, wobei $R_3$ die im allgemeinen Anspruch beschriebenen Bedeutungen hat, werden dadurch erhalten, daß man Fumarsäuremonochloridmonoester-Derivate der Formel II

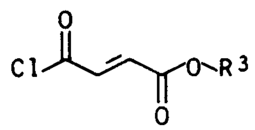
II

beispielsweise in Gegenwart geeigneter Reaktionshilfsmittel mit Imidoestern III

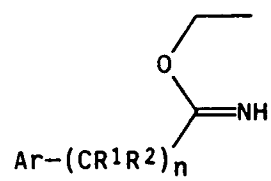

beispielsweise bei Temperaturen zwischen -40 und +10°C, bevorzugt -10 bis 0°C, beispielsweise in einem geeigneten Verdünnungsmittel zur Reaktion bringt. Als Verdünnungsmittel kommen polare inerte Lösungsmittel wie Ether, z.B. Tetrahydrofuran, 1,4-Dioxan, Ketone, insbesondere Aceton, Methylethylketon, Nitrile, z. B. Acetonitril, Propionitril, Amide, vor allem N,N-Dimethylformamid, N-Methylpyrrolidon, N,N-Dimethylethylenharnstoff oder N,N-Dimethylpropylenharnstoff, Sulfoxide, besonders Dimethylsulfoxid, in Frage.

Als Reaktionshilfsmittel eignen sich alle gängigen tertiären Amine, insbesondere Triethylamin, N,N-Dimethylanilin, Piperidin.

Die Darstellung der Benzylimidoester III ist literaturbekannt (siehe z.B. J. org. Chem. 30, 699 (1965)), ebenso sind die Fumarsäuremonochloridemonoester durch ein bekanntes Verfahren zugänglich. (J. Med. Chem., 29, 1868 (1986)).

Verbindungen der allgemeinen Formel 1 mit A = $NR^4R^5$, wobei $R^4R^5$ die im allgemeinen Anspruch formulierten Bedeutungen haben, sind dadurch zugänglich, daß man Fumaramidsäure IV

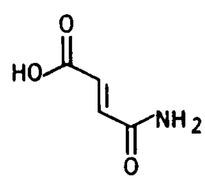

beispielsweise in einem geeigneten Verdünnungsmittel beispielsweise in Gegenwart eines Reaktionshilfsmittels beispielsweise bei Temperaturen zwischen -60 bis -10°C mit Chlorameisensäureethylester zur Reaktion bringt und danach mit dem Imidoester III beispielsweise bei Temperaturen zwischen -40 bis +10°C umsetzt.

Als Verdünnungsmittel kommen polare inerte Lösungsmittel wie Ether, z.B. Tetrahydrofuran, 1,4-Dioxan, Ketone, insbesondere Aceton, Methylethylketon, Nitrile, z. B. Acetonitril, Propionitril, Amide, vor allem N,N-Dimethylformamid, N-Methylpyrrolidon, N,N-Dimethylethylenharnstoff oder N,N-Dimethylpropylenharnstoff, Sulfoxide, besonders Dimethylsulfoxid, Piperidin in Frage.

Die Verbindung 193 - N-(3-Chlor-4-hydroxy-phenylacetyl)furmarsäuremethylester - kann wie oben beschrieben hergestellt werden. Sie kann aber auch durch Kultivierung des Pilzes DSM Nr. 6218 und Abtrennung aus dem Kulturfiltrat hergestellt werden.

Der Pilz - eine Coniothyrium-Art wurde aus einer Gartenerdprobe aus Bamako, Mali isoliert.

Eine lebensfähige Kultur dieses Mikroorganismus wurde bei der Deutschen Sammlung von Mikroorganismen und Zellkulturen GmbH, D-3300 Braunschweig, hinterlegt und in deren Sammlung aufgenommen. Der Mikroorganismus ist der Öffentlichkeit bei dieser Hinterlegungsstelle unter der Bezeichnung DSM Nr.

6218 frei zugänglich.

Die Verbindung 193 wird - allgemein gesprochen - dadurch hergestellt, daß man den Pilz DSM Nr. 6218 unter aneroben Bedingungen in einem flüssigen Medium, enthaltend assimilierbare Quellen an Kohlenstoff, Stickstoff und anorganischen Anionen und Kationen, kultiviert, bis sich eine wesentliche Menge der Verbindung Nr. 193 in dem Medium gebildet hat und nachfolgend diese Verbindung daraus gewinnt.

Die Verbindung Nr. 193 wird beispielsweise hergestellt, indem man unter aeroben Bedingungen ein flüssiges Medium fermentiert, das assimilierbare Quellen an Kohlenstoff, Stickstoff und anorganischen Anionen und Kationen enthält, wobei das Medium mit einer lebensfähigen Kultur des Pilzes DSM Nr. 6218 oder einer die Verbindung Nr. 193 produzierenden Mutante desselben beimpft wurde, den Organismus bei steriler Belüftung und unter Rühren während eines Zeitraumes von 50 bis 400 Stunden bei einer Temperatur von 20 bis 30°C und pH 5,5 bis 8,0 kultiviert, das Kulturfiltrat abtrennt und die Verbindung daraus extrahiert.

Die Extraktion der Verbindung Nr. 193 kann beispielsweise in der Weise erfolgen, daß man sie aus dem Kulturfiltrat mit einem mit Wasser nicht mischbaren Lösemittel oder nach Gefriertrocknung des Kulturfiltrates mit einem Lösemittel aus diesem extrahiert.

Die Reinigung des Extraktes kann beispielsweise durch chromatographische Verfahren erfolgen. Für die Anwendung als Fungizid ist nicht nur die VerbindungNr. 193 als solche, sondern auch die Fermentationsbrühe oder Gesamtmaische des Mikroorganismus geeignet. Man kann auch den aus der Fermentationsbrühe oder Gesamtmaische des Mikroorganismus 193 gewonnenen Extrakt verwenden. Die Verbindung Nr. 193 entsteht im Verlauf der Kultivierung des Pilzes DSM Nr. 6218 unter kontrollierten Bedingungen.

Allgemeine Fermentationsbedingungen

Die Kultivierung des Pilzes DSM Nr. 6218 kann in einer breiten Vielfalt flüssiger Kulturmedien durchgeführt werden. Geeignete Nährmedien umfassen eine assimilierbare Quelle von Kohlenstoff, wie Dextrin, Saccharose, Melasse, Glycerin, etc.; eine assimilierbare Quelle an Stickstoff, wie Protein, Proteinhydrolysat, Polypeptide, Aminosäuren, Maisquellwasser, etc.; sowie anorganische Anionen und Kationen, wie Kalium, Natrium, Ammonium, Calcium, Sulfat, Carbonat, Phosphat, Chlorid, etc. Spurenelemente wie Bor, Molybdän, Kupfer, Mangan, Zink, Eisen, etc. werden in Form von Verunreinigungen der anderen Bestandteile der Medien oder als definierte Lösungen den Medien zugeführt. Die Belüftung in Flaschen und Tanks wird durchgeführt, indem man sterile Luft durch das Nährmedium hindurch leitet oder auf die Oberfläche des Nährmediums aufpreßt. Eine weitere Bewegung in den Tanks wird durch einen mechanischen Rührer gewährleistet. Falls erforderlich kann ein Antischum-Mittel wie Siliconöl, zugesetzt werden.

Beispiel a

Inoculum-Herstellung

Geeignete Medien, wie sie zur Anzucht des Inoculums verwendet werden, sind feste sowie flüssige Medien, wie Kartoffel-Karotten-Agar, synthetic nutrient agar, nutrient broth agar, Kartoffel-Saccharose-Agar, Malzextrakt-Pepton-Hefeextrakt-Agar und Biomalz-Agar (jeweils 2 %ig).

aa) Für die Fermentation in kleinem Maßstab

Die Nährmedien werden sterilisiert. Der gleichmäßig mit der keimfreien Kultur des Pilzes bewachsene Agar (2 % Malz-Extrakt) von 5 Glasschalen (9 cm = $\phi$) wird in einem sterilisierten Mixer unter Zusatz von 200 ml Wasser sterilisiertem Wasser (entionisiert) homogenisiert und anschließend jeweils 40 ml dieses Homogenisates als Inokulum für die Kultur des Pilzes in Glaskolben (jeweils 500 ml Nährmedium) verwendet.

ab) Für die Fermentation in größerem Maßstab

Die Nährmedien werden sterilisiert. Die gleichmäßig mit der keimfreien Kultur des Pilzes DSM Nr. 6218 bewachsene Agar (2 % Malz-Extrakt) von 5 Glasschalen (9 cm $\phi$) wird in einem sterilisierten Mixer unter Zusatz von 400 ml sterilisiertem Wasser (entionisiert) homogenisiert und dann jeweils die Hälfte dieses Homogenisates als Inokulum in 1 l Glaskolben (mit jeweils 500 ml Nährmedium) überführt. Die Medien werden anschließend 24 - 30 h bei 25°C auf einem Rotationsschüttler (130 bis 150 U/min) geschüttelt. Dieses Inokulum wird anschließend verwendet, um 5 l eines sterilen Malzextrat-Mediums (2 %) zu

beimpfen.

Beispiel b

Fermentation in kleinem Maßstab

Zur Bereitung der Fermentationsmedien wurde Biomalz-Medium (5 %) verwendet. Nach dem Beimpfen der sterilisierten Medien (jeweils 500 ml Nährmedium pro Kolben) mit dem nach Beispiel aa hergestellten Inokulum werden diese anschließend 5 bis 16 Tage bei 25 °C auf einem Rotationsschüttler (130 bis 150 U/min) geschüttelt und dann die Maische geerntet.

Beispiel C

Fermentation in größerem Maßstab

Zur Bereitung der Fermentationsmedien wurde Malzextrat-Medium (2 %) verwendet. Nach dem Beimpfen des sterilisierten Mediums (15 l Nährmedium) mit dem nach Beispiel ab hergestellten Inokulum wird die Fermentation bei 25 °C wobei ein steriler Luftstrom von 1,5 bis 2 l/min aufrechterhalten und unter Rühren mit einem Blattrührer, der mit 150 U/min betrieben wird, durchgeführt. Die Fermentation wird 10 - 14 Tage betrieben und anschließend die Maische geerntet. Analog diesem Beispiel werden Fermentationen im 50 l- bzw. 100 l-Maßstab durchgeführt, wobei die Mengean Inokulum und der sterile Luftstrom den geänderten Volumina an Nährmedium entsprechend angepaßt werden.

Beispiel d

Isolierung von Verbindung Nr. 193

Ein Gesamtvolumen von 10 l Kulturbrühe hergestellt in Biomalz-Schüttelkulturen, wird filtriert und der Mycelkuchen verworfen, da er keine biologische Aktivität aufweist. Das so erhaltene Kulturfiltrat wird mit frisch destilliertem Ethylacetat extrahiert und aus diesem Extrakt anschließend am Rotationsverdampfer unter Vakuum das Lösungsmittel entfernt. Der Rückstand wird in einem geringen Volumen Aceton aufgenommen, über Natriumsulfat filtriert und anschließend einer Dickschichtchromatographie unterworfen.

Bedingungen:

| | |
|---|---|
| stationäre Phase: | Siliciumdioxid PF 254/366, Schichtdicke = 1,25 mm (Fa. Merck, Darmstadt) |
| mobile Phase: | Dichlormethan : Methanol = 95 : 5 |
| Detektion der Fraktion: | = 254 nm |
| RF-Wert der Fraktion: | 0,64 |

Die den Hauptanteil an Verbindung 193 enthaltende Fraktion wird mit einem Gemisch von Dichlormethan : Diethylether 90 : 10 vom Kieselgel eluiert und am Rotationsverdampfer unter Vakuum zur Trockene eingeengt. Man nimmt in heißem Aceton auf und nach einiger Zeit kristallisiert die Verbindung 193 aus. Durch nochmaliges Umkristallieren erhält man ein sehr reines Produkt vom Schmelzpunkt 153 °C (Auswaage: 305 mg).

Die folgenden Beispiele erläutern die Herstellung der neuen Verbindungen.

Beispiel 1

N-(p-Methoxyphenylacetyl)-fumarsäurediamid

0,2 g (1,73 mmol) Fumaramidsäure werden mit 0,24 ml (1,73 mmol) Triethylamin versetzt und in 2,8 ml trockenem N,N-Dimethylformamid (DMF) gelöst. Man kühlt auf -20°C und gibt die Lösung von 0,164 ml (1,73 mmol) Chlorameisensäureethylester in 1 ml trockenem Diethylether tropfenweise zu. Man läßt weitere 45 min bei -20° rühren und gibt dann schnell eine -20°C kalte Lösung von 0,398 g (1,73 mmol) p-Methoxyphenylessigsäureimidoethylester-Hydrochlorid in 2 ml trockenem N,N-Dimethylformamid und 0,24 ml (1,73 mmol) Triethylamin zu. Man läßt 3 h bei -10 bis 0°C rühren, filtriert vom Feststoff ab, wäscht diesen mit N,N-Dimethylformamid und zieht das Lösungsmittel im Hochvakuum bei maximal 40° ab. Der Rückstand wird in 2 ml Wasser aufgenommen, auf 0°C gekühlt und mit 1 n Salzsäure auf pH = 1,5 gebracht.

Man läßt 30 min rühren, saugt den Feststoff ab und kristallisiert zweimal aus Dichlormethan um. Man erhäl 0,201 g (44 %) des Diamids.

Beispiel 2

N-(p-Methylphenylacetyl)-fumaramidsäureethylester (Verbindung Nr. 224)

8,3 g (0,05 mol) Fumarsäuremonoethylestermonochlorid und 5,05 g (0,05 mol) Triethylamin werden in 50 ml trockenem DMF gelöst und auf -20°C gekühlt. Dazu werden schnell 10,7 g (0,05 mol) p-Methylphenylessigsäureimidoethylester-Hydrochlorid und 5,05 g (0,05 mol) Triethylamin in 50 ml trockenem DMF getropft und 3 h bei einer Temperatur von -10 bis 0°C gerührt. Anschließend läßt man auf Raumtemperatur kommen, saugt vom Feststoff ab, wäscht mit wenig DMF nach und destilliert das Lösungsmittel bei maximal 40°C im Vakuum ab. Der Rückstand wird in 250 ml Wasser aufgenommen, auf 0°C gekühlt, mit Salzsäure auf pH = 1,5 gebracht und 30 min bei dieser Temperatur gerührt. Der Feststoff wird abgesaugt und aus Diethylether umkristallisiert. Man erhält 3,1 g (22,5 %) der Titelverbindung.

In entsprechender Weise können die in der folgenden Tabelle angeführten Verbindungen hergestellt werden.

Tabelle

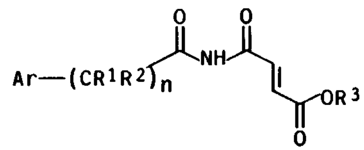

| Nr. | n | $R^1$ | $R^2$ | $R^3$ | Ar | physik. Daten IR ($cm^{-1}$) |
|---|---|---|---|---|---|---|
| 1 | 0 | - | - | $CH_3$ | Phenyl | |
| 2 | 0 | - | - | $CH_3$ | 2-Methylphenyl | |
| 3 | 0 | - | - | $CH_3$ | 3-Methylphenyl | |
| 4 | 0 | - | - | $CH_3$ | 4-Methylphenyl | |
| 5 | 0 | - | - | $CH_3$ | 2,4-Dimethylphenyl | |
| 6 | 0 | - | - | $CH_3$ | 2,6-Dimethylphenyl | |
| 7 | 0 | - | - | $CH_3$ | 2,4,6-Trimethylphenyl | |
| 8 | 0 | - | - | $CH_3$ | 4-tert.-Butylphenyl | |
| 9 | 0 | - | - | $CH_3$ | 1-Naphthyl | |
| 10 | 0 | - | - | $CH_3$ | 2-Naphthyl | |
| 11 | 0 | - | - | $CH_3$ | 4-Biphenylyl | |
| 12 | 0 | - | - | $CH_3$ | 2-Fluorphenyl | |
| 13 | 0 | - | - | $CH_3$ | 3-Fluorphenyl | |
| 14 | 0 | - | - | $CH_3$ | 4-Fluorphenyl | |
| 15 | 0 | - | - | $CH_3$ | 2,4-Difluorphenyl | |
| 16 | 0 | - | - | $CH_3$ | 2-Chlorphenyl | |
| 17 | 0 | - | - | $CH_3$ | 3-Chlorphenyl | |
| 18 | 0 | - | - | $CH_3$ | 4-Chlorphenyl | |

Fortsetzung Tabelle

| Nr. | n | $R^1$ | $R^2$ | $R^3$ | Ar | physik. Daten IR ($cm^{-1}$) |
|---|---|---|---|---|---|---|
| 19 | 0 | - | - | $CH_3$ | 2,4-Dichlorphenyl | |
| 20 | 0 | - | - | $CH_3$ | 3,4-Dichlorphenyl | |
| 21 | 0 | - | - | $CH_3$ | 2,6-Dichlorphenyl | |
| 22 | 0 | - | - | $CH_3$ | 2,4,6-Trichlorphenyl | |
| 23 | 0 | - | - | $CH_3$ | 2-Chlor-3-fluorphenyl | |
| 24 | 0 | - | - | $CH_3$ | 2-Chlor-4-fluorphenyl | |
| 25 | 0 | - | - | $CH_3$ | 2-Chlor-4-fluorphenyl | |
| 26 | 0 | - | - | $CH_3$ | 3-Chlor-4-methoxyphenyl | |
| 27 | 0 | - | - | $CH_3$ | 2-Chlor-4-methylphenyl | |
| 28 | 0 | - | - | $CH_3$ | 3-Chlor-4-hydroxyphenyl | |
| 29 | 0 | - | - | $CH_3$ | 2-Chlor-6-fluorphenyl | |
| 30 | 0 | - | - | $CH_3$ | 3-Fluor-4-methoxyphenyl | |
| 31 | 0 | - | - | $CH_3$ | 2-Bromphenyl | |
| 32 | 0 | - | - | $CH_3$ | 3-Bromphenyl | |
| 33 | 0 | - | - | $CH_3$ | 4-Bromphenyl | |
| 34 | 0 | - | - | $CH_3$ | 2-Brom-4-fluorphenyl | |
| 35 | 0 | - | - | $CH_3$ | 2-Brom-4-chlorphenyl | |
| 36 | 0 | - | - | $CH_3$ | 2-Methoxyphenyl | |
| 37 | 0 | - | - | $CH_3$ | 3-Methoxyphenyl | |
| 38 | 0 | - | - | $CH_3$ | 4-Methoxyphenyl | |
| 39 | 0 | - | - | $CH_3$ | 4-Butoxyphenyl | |
| 40 | 0 | - | - | $CH_3$ | 4-n-Propoxyphenyl | |
| 41 | 0 | - | - | $CH_3$ | 4-tert-.Butoxyphenyl | |
| 42 | 0 | - | - | $CH_3$ | 2-Trifluormethylphenyl | |

Fortsetzung Tabelle

| Nr. | n | $R^1$ | $R^2$ | $R^3$ | Ar | physik. Daten IR ($cm^{-1}$) |
|---|---|---|---|---|---|---|
| 43 | 0 | - | - | $CH_3$ | 3-Trifluormethylphenyl | |
| 44 | 0 | - | - | $CH_3$ | 4-Trifluormethylphenyl | |
| 45 | 0 | - | - | $CH_3$ | 4-Trifluormethoxyphenyl | |
| 46 | 0 | - | - | $CH_3$ | 4-Tetrafluorethoxyphenyl | |
| 47 | 0 | - | - | $CH_3$ | 3,4-Dimethoxyphenyl | |
| 48 | 0 | - | - | $CH_3$ | 2-Methoxy-4-trifluormethylphenyl | |
| 49 | 0 | - | - | $CH_3$ | 4-Phenoxyphenyl | |
| 50 | 0 | - | - | $CH_3$ | 4-(4'-Chlorphenoxy)-phenyl | |
| 51 | 0 | - | - | $CH_3$ | 2-Nitrophenyl | |
| 52 | 0 | - | - | $CH_3$ | 4-Nitrophenyl | |
| 53 | 0 | - | - | $CH_3$ | 2-Chlor-4-nitrophenyl | |
| 54 | 0 | - | - | $CH_3$ | 4-Hydroxyphenyl | |
| 55 | 0 | - | - | $CH_3$ | 2-Hydroxyphenyl | |
| 56 | 0 | - | - | $C_2H_5$ | Phenyl | |
| 57 | 0 | - | - | $C_2H_5$ | 2-Methylphenyl | |
| 58 | 0 | - | - | $C_2H_5$ | 3-Methylphenyl | |
| 59 | 0 | - | - | $C_2H_5$ | 4-Methylphenyl | |
| 60 | 0 | - | - | $C_2H_5$ | 2,4-Dimethylphenyl | |
| 61 | 0 | - | - | $C_2H_5$ | 2,6-Dimethylphenyl | |
| 62 | 0 | - | - | $C_2H_5$ | 2,4,6-Trimethylphenyl | |
| 63 | 0 | - | - | $C_2H_5$ | 4-tert.-Butylphenyl | |
| 64 | 0 | - | - | $C_2H_5$ | 1-Naphthyl | |
| 65 | 0 | - | - | $C_2H_5$ | 2-Naphthyl | |
| 66 | 0 | - | - | $C_2H_5$ | 4-Biphenylyl | |

Fortsetzung Tabelle

| Nr. | n | $R^1$ | $R^2$ | $R^3$ | Ar | physik. Daten IR ($cm^{-1}$) |
|---|---|---|---|---|---|---|
| 67 | 0 | - | - | $C_2H_5$ | 2-Fluorphenyl | |
| 68 | 0 | - | - | $C_2H_5$ | 3-Fluorphenyl | |
| 69 | 0 | - | - | $C_2H_5$ | 4-Fluorphenyl | |
| 70 | 0 | - | - | $C_2H_5$ | 2,4-Difluorphenyl | |
| 71 | 0 | - | - | $C_2H_5$ | 2-Chlorphenyl | |
| 72 | 0 | - | - | $C_2H_5$ | 3-Chlorphenyl | |
| 73 | 0 | - | - | $C_2H_5$ | 4-Chlorphenyl | |
| 74 | 0 | - | - | $C_2H_5$ | 2,4-Dichlorphenyl | |
| 75 | 0 | - | - | $C_2H_5$ | 3,4-Dichlorphenyl | |
| 76 | 0 | - | - | $C_2H_5$ | 2,6-Dichlorphenyl | |
| 77 | 0 | - | - | $C_2H_5$ | 2,4,6-Trichlorphenyl | |
| 78 | 0 | - | - | $C_2H_5$ | 2-Chlor-3-fluorphenyl | |
| 79 | 0 | - | - | $C_2H_5$ | 2-Chlor-4-fluorphenyl | |
| 80 | 0 | - | - | $C_2H_5$ | 2-Chlor-4-fluorphenyl | |
| 81 | 0 | - | - | $C_2H_5$ | 3-Chlor-4-methoxyphenyl | |
| 82 | 0 | - | - | $C_2H_5$ | 2-Chlor-4-methylphenyl | |
| 83 | 0 | - | - | $C_2H_5$ | 3-Chlor-4-hydroxyphenyl | |
| 84 | 0 | - | - | $C_2H_5$ | 2-Chlor-6-fluorphenyl | |
| 85 | 0 | - | - | $C_2H_5$ | 3-Fluor-4-methoxyphenyl | |
| 86 | 0 | - | - | $C_2H_5$ | 2-Bromphenyl | |
| 87 | 0 | - | - | $C_2H_5$ | 3-Bromphenyl | |
| 88 | 0 | - | - | $C_2H_5$ | 4-Bromphenyl | |
| 89 | 0 | - | - | $C_2H_5$ | 2-Brom-4-fluorphenyl | |
| 90 | 0 | - | - | $C_2H_5$ | 2-Brom-4-chlorphenyl | |

Fortsetzung Tabelle

| Nr. | n | $R^1$ | $R^2$ | $R^3$ | Ar | physik. Daten IR ($cm^{-1}$) |
|---|---|---|---|---|---|---|
| 91 | 0 | - | - | $C_2H_5$ | 2-Methoxyphenyl | |
| 92 | 0 | - | - | $C_2H_5$ | 3-Methoxyphenyl | |
| 93 | 0 | - | - | $C_2H_5$ | 4-Methoxyphenyl | |
| 94 | 0 | - | - | $C_2H_5$ | 4-Butoxyphenyl | |
| 95 | 0 | - | - | $C_2H_5$ | 4-n-Propoxyphenyl | |
| 96 | 0 | - | - | $C_2H_5$ | 4-tert-.Butoxyphenyl | |
| 97 | 0 | - | - | $C_2H_5$ | 2-Trifluormethylphenyl | |
| 98 | 0 | - | - | $C_2H_5$ | 3-Trifluormethylphenyl | |
| 99 | 0 | - | - | $C_2H_5$ | 4-Trifluormethylphenyl | |
| 100 | 0 | - | - | $C_2H_5$ | 4-Trifluormethoxyphenyl | |
| 101 | 0 | - | - | $C_2H_5$ | 4-Tetrafluorethoxyphenyl | |
| 102 | 0 | - | - | $C_2H_5$ | 3,4-Dimethoxyphenyl | |
| 103 | 0 | - | - | $C_2H_5$ | 2-Methoxy-4-trifluormethylphenyl | |
| 104 | 0 | - | - | $C_2H_5$ | 4-Phenoxyphenyl | |
| 105 | 0 | - | - | $C_2H_5$ | 4-(4'-Chlorphenoxy)-phenyl | |
| 106 | 0 | - | - | $C_2H_5$ | 2-Nitrophenyl | |
| 107 | 0 | - | - | $C_2H_5$ | 4-Nitrophenyl | |
| 108 | 0 | - | - | $C_2H_5$ | 2-Chlor-4-nitrophenyl | |
| 109 | 0 | - | - | $C_2H_5$ | 4-Hydroxyphenyl | |
| 110 | 0 | - | - | $C_2H_5$ | 2-Hydroxyphenyl | |
| 111 | 0 | - | - | iso-$C_3H_7$ | Phenyl | |
| 112 | 0 | - | - | iso-$C_3H_7$ | 2-Methylphenyl | |
| 113 | 0 | - | - | iso-$C_3H_7$ | 3-Methylphenyl | |
| 114 | 0 | - | - | iso-$C_3H_7$ | 4-Methylphenyl | |

Fortsetzung Tabelle

| Nr. | n | $R^1$ | $R^2$ | $R^3$ | Ar | physik. Daten IR ($cm^{-1}$) |
|---|---|---|---|---|---|---|
| 115 | 0 | - | - | iso-$C_3H_7$ | 2,4-Dimethylphenyl | |
| 116 | 0 | - | - | iso-$C_3H_7$ | 2,6-Dimethylphenyl | |
| 117 | 0 | - | - | iso-$C_3H_7$ | 2,4,6-Trimethylphenyl | |
| 118 | 0 | - | - | iso-$C_3H_7$ | 4-tert.-Butylphenyl | |
| 119 | 0 | - | - | iso-$C_3H_7$ | 1-Naphthyl | |
| 120 | 0 | - | - | iso-$C_3H_7$ | 2-Naphthyl | |
| 121 | 0 | - | - | iso-$C_3H_7$ | 4-Biphenyl | |
| 122 | 0 | - | - | iso-$C_3H_7$ | 2-Fluorphenyl | |
| 123 | 0 | - | - | iso-$C_3H_7$ | 3-Fluorphenyl | |
| 124 | 0 | - | - | iso-$C_3H_7$ | 4-Fluorphenyl | |
| 125 | 0 | - | - | iso-$C_3H_7$ | 2,4-Difluorphenyl | |
| 126 | 0 | - | - | iso-$C_3H_7$ | 2-Chlorphenyl | |
| 127 | 0 | - | - | iso-$C_3H_7$ | 3-Chlorphenyl | |
| 128 | 0 | - | - | iso-$C_3H_7$ | 4-Chlorphenyl | |
| 129 | 0 | - | - | iso-$C_3H_7$ | 2,4-Dichlorphenyl | |
| 130 | 0 | - | - | iso-$C_3H_7$ | 3,4-Dichlorphenyl | |
| 131 | 0 | - | - | iso-$C_3H_7$ | 2,6-Dichlorphenyl | |
| 132 | 0 | - | - | iso-$C_3H_7$ | 2,4,6-Trichlorphenyl | |
| 133 | 0 | - | - | iso-$C_3H_7$ | 2-Chlor-3-fluorphenyl | |
| 134 | 0 | - | - | iso-$C_3H_7$ | 2-Chlor-4-fluorphenyl | |
| 135 | 0 | - | - | iso-$C_3H_7$ | 2-Chlor-4-fluorphenyl | |
| 136 | 0 | - | - | iso-$C_3H_7$ | 3-Chlor-4-methoxyphenyl | |
| 137 | 0 | - | - | iso-$C_3H_7$ | 2-Chlor-4-methylphenyl | |
| 138 | 0 | - | - | iso-$C_3H_7$ | 3-Chlor-4-hydroxyphenyl | |

Fortsetzung Tabelle

| Nr. | n | $R^1$ | $R^2$ | $R^3$ | Ar | physik. Daten IR ($cm^{-1}$) |
|---|---|---|---|---|---|---|
| 139 | 0 | - | - | iso-$C_3H_7$ | 2-Chlor-6-fluorphenyl | |
| 140 | 0 | - | - | iso-$C_3H_7$ | 3-Fluor-4-methoxyphenyl | |
| 141 | 0 | - | - | iso-$C_3H_7$ | 2-Bromphenyl | |
| 142 | 0 | - | - | iso-$C_3H_7$ | 3-Bromphenyl | |
| 143 | 0 | - | - | iso-$C_3H_7$ | 4-Bromphenyl | |
| 144 | 0 | - | - | iso-$C_3H_7$ | 2-Brom-4-fluorphenyl | |
| 145 | 0 | - | - | iso-$C_3H_7$ | 2-Brom-4-chlorphenyl | |
| 146 | 0 | - | - | iso-$C_3H_7$ | 2-Methoxyphenyl | |
| 147 | 0 | - | - | iso-$C_3H_7$ | 3-Methoxyphenyl | |
| 148 | 0 | - | - | iso-$C_3H_7$ | 4-Methoxyphenyl | |
| 149 | 0 | - | - | iso-$C_3H_7$ | 4-Butoxyphenyl | |
| 150 | 0 | - | - | iso-$C_3H_7$ | 4-n-Propoxyphenyl | |
| 151 | 0 | - | - | iso-$C_3H_7$ | 4-tert-.Butoxyphenyl | |
| 152 | 0 | - | - | iso-$C_3H_7$ | 2-Trifluormethylphenyl | |
| 153 | 0 | - | - | iso-$C_3H_7$ | 3-Trifluormethylphenyl | |
| 154 | 0 | - | - | iso-$C_3H_7$ | 4-Trifluormethylphenyl | |
| 155 | 0 | - | - | iso-$C_3H_7$ | 4-Trifluormethoxyphenyl | |
| 156 | 0 | - | - | iso-$C_3H_7$ | 4-Tetrafluorethoxyphenyl | |
| 157 | 0 | - | - | iso-$C_3H_7$ | 3,4-Dimethoxyphenyl | |
| 158 | 0 | - | - | iso-$C_3H_7$ | 2-Methoxy-4-trifluormethylphenyl | |
| 159 | 0 | - | - | iso-$C_3H_7$ | 4-Phenoxyphenyl | |
| 160 | 0 | - | - | iso-$C_3H_7$ | 4-(4'-Chlorphenoxy)-phenyl | |
| 161 | 0 | - | - | iso-$C_3H_7$ | 2-Nitrophenyl | |
| 162 | 0 | - | - | iso-$C_3H_7$ | 4-Nitrophenyl | |

Fortsetzung Tabelle

| Nr. | n | $R^1$ | $R^2$ | $R^3$ | Ar | physik. Daten IR ($cm^{-1}$) |
|---|---|---|---|---|---|---|
| 163 | 0 | - | - | iso-$C_3H_7$ | 2-Chlor-4-nitrophenyl | |
| 164 | 0 | - | - | iso-$C_3H_7$ | 4-Hydroxyphenyl | |
| 165 | 0 | - | - | iso-$C_3H_7$ | 2-Hydroxyphenyl | |
| 166 | 1 | H | H | $CH_3$ | Phenyl | Fp. 135° |
| 167 | 1 | H | H | $CH_3$ | 2-Methylphenyl | |
| 168 | 1 | H | H | $CH_3$ | 3-Methylphenyl | |
| 169 | 1 | H | H | $CH_3$ | 4-Methylphenyl | |
| 170 | 1 | H | H | $CH_3$ | 2,4-Dimethylphenyl | |
| 171 | 1 | H | H | $CH_3$ | 2,6-Dimethylphenyl | |
| 172 | 1 | H | H | $CH_3$ | 2,4,6-Trimethylphenyl | |
| 173 | 1 | H | H | $CH_3$ | 4-tert.-Butylphenyl | |
| 174 | 1 | H | H | $CH_3$ | 1-Naphthyl | |
| 175 | 1 | H | H | $CH_3$ | 2-Naphthyl | |
| 176 | 1 | H | H | $CH_3$ | 4-Biphenyl | |
| 177 | 1 | H | H | $CH_3$ | 2-Fluorphenyl | |
| 178 | 1 | H | H | $CH_3$ | 3-Fluorphenyl | |
| 179 | 1 | H | H | $CH_3$ | 4-Fluorphenyl | |
| 180 | 1 | H | H | $CH_3$ | 2,4-Difluorphenyl | |
| 181 | 1 | H | H | $CH_3$ | 2-Chlorphenyl | |
| 182 | 1 | H | H | $CH_3$ | 3-Chlorphenyl | |
| 183 | 1 | H | H | $CH_3$ | 4-Chlorphenyl | Fp. 153 |
| 184 | 1 | H | H | $CH_3$ | 2,4-Dichlorphenyl. | |
| 185 | 1 | H | H | $CH_3$ | 3,4-Dichlorphenyl | |
| 186 | 1 | H | H | $CH_3$ | 2,6-Dichlorphenyl | |

Fortsetzung Tabelle

| Nr. | n | $R^1$ | $R^2$ | $R^3$ | Ar | physik. Daten IR ($cm^{-1}$) |
|---|---|---|---|---|---|---|
| 187 | 1 | H | H | $CH_3$ | 2,4,6-Trichlorphenyl | |
| 188 | 1 | H | H | $CH_3$ | 2-Chlor-3-fluorphenyl | |
| 189 | 1 | H | H | $CH_3$ | 2-Chlor-4-fluorphenyl | |
| 190 | 1 | H | H | $CH_3$ | 2-Chlor-4-fluorphenyl | |
| 191 | 1 | H | H | $CH_3$ | 3-Chlor-4-methoxyphenyl | |
| 192 | 1 | H | H | $CH_3$ | 2-Chlor-4-methylphenyl | |
| 193 | 1 | H | H | $CH_3$ | 3-Chlor-4-hydroxyphenyl | Fp.: 153° |
| 194 | 1 | H | H | $CH_3$ | 2-Chlor-6-fluorphenyl | |
| 195 | 1 | H | H | $CH_3$ | 3-Fluor-4-methoxyphenyl | |
| 196 | 1 | H | H | $CH_3$ | 2-Bromphenyl | |
| 197 | 1 | H | H | $CH_3$ | 3-Bromphenyl | |
| 198 | 1 | H | H | $CH_3$ | 4-Bromphenyl | |
| 199 | 1 | H | H | $CH_3$ | 2-Brom-4-fluorphenyl | |
| 200 | 1 | H | H | $CH_3$ | 2-Brom-4-chlorphenyl | |
| 201 | 1 | H | H | $CH_3$ | 2-Methoxyphenyl | |
| 202 | 1 | H | H | $CH_3$ | 3-Methoxyphenyl | |
| 203 | 1 | H | H | $CH_3$ | 4-Methoxyphenyl | Fp.: 142° |
| 204 | 1 | H | H | $CH_3$ | 4-Butoxyphenyl | Fp.: 184° |
| 205 | 1 | H | H | $CH_3$ | 4-n-Propoxyphenyl | |
| 206 | 1 | H | H | $CH_3$ | 4-tert-.Butoxyphenyl | |
| 207 | 1 | H | H | $CH_3$ | 2-Trifluormethylphenyl | |
| 208 | 1 | H | H | $CH_3$ | 3-Trifluormethylphenyl | |
| 209 | 1 | H | H | $CH_3$ | 4-Trifluormethylphenyl | |
| 210 | 1 | H | H | $CH_3$ | 4-Trifluormethoxyphenyl | |

Fortsetzung Tabelle

| Nr. | n | $R^1$ | $R^2$ | $R^3$ | Ar | physik. Daten IR ($cm^{-1}$) |
|---|---|---|---|---|---|---|
| 211 | 1 | H | H | $CH_3$ | 4-Tetrafluorethoxyphenyl | |
| 212 | 1 | H | H | $CH_3$ | 3,4-Dimethoxyphenyl | |
| 213 | 1 | H | H | $CH_3$ | 2-Methoxy-4-trifluormethylphenyl | |
| 214 | 1 | H | H | $CH_3$ | 4-Phenoxyphenyl | |
| 215 | 1 | H | H | $CH_3$ | 4-(4'-Chlorphenoxy)-phenyl | |
| 216 | 1 | H | H | $CH_3$ | 2-Nitrophenyl | |
| 217 | 1 | H | H | $CH_3$ | 4-Nitrophenyl | |
| 218 | 1 | H | H | $CH_3$ | 2-Chlor-4-nitrophenyl | |
| 219 | 1 | H | H | $CH_3$ | 4-Hydroxyphenyl | Fp.: 205° |
| 220 | 1 | H | H | $CH_3$ | 2-Hydroxyphenyl | |
| 222 | 1 | H | H | $C_2H_5$ | 2-Methylphenyl | |
| 223 | 1 | H | H | $C_2H_5$ | 3-Methylphenyl | |
| 224 | 1 | H | H | $C_2H_5$ | 4-Methylphenyl | 3280, 1737, 1688, 1371, 1318, 1171 Fp: 91 |
| 225 | 1 | H | H | $C_2H_5$ | 2,4-Dimethylphenyl | |
| 226 | 1 | H | H | $C_2H_5$ | 2,6-Dimethylphenyl | |
| 227 | 1 | H | H | $C_2H_5$ | 2,4,6-Trimethylphenyl | |
| 228 | 1 | H | H | $C_2H_5$ | 4-tert.-Butylphenyl | |
| 229 | 1 | H | H | $C_2H_5$ | 1-Naphthyl | |
| 230 | 1 | H | H | $C_2H_5$ | 2-Naphthyl | |
| 231 | 1 | H | H | $C_2H_5$ | 4-Biphenyl | |
| 232 | 1 | H | H | $C_2H_5$ | 2-Fluorphenyl | |
| 233 | 1 | H | H | $C_2H_5$ | 3-Fluorphenyl | |

Fortsetzung Tabelle

| Nr. | n | $R^1$ | $R^2$ | $R^3$ | Ar | physik. Daten IR ($cm^{-1}$) |
|---|---|---|---|---|---|---|
| 234 | 1 | H | H | $C_2H_5$ | 4-Fluorphenyl | 3200, 1738, 1689, 1576, 1510, 1244 Fp: 102 |
| 235 | 1 | H | H | $C_2H_5$ | 2,4-Difluorphenyl | |
| 236 | 1 | H | H | $C_2H_5$ | 2-Chlorphenyl | |
| 237 | 1 | H | H | $C_2H_5$ | 3-Chlorphenyl | |
| 238 | 1 | H | H | $C_2H_5$ | 4-Chlorphenyl | |
| 239 | 1 | H | H | $C_2H_5$ | 2,4-Dichlorphenyl | |
| 240 | 1 | H | H | $C_2H_5$ | 3,4-Dichlorphenyl | |
| 241 | 1 | H | H | $C_2H_5$ | 2,6-Dichlorphenyl | |
| 242 | 1 | H | H | $C_2H_5$ | 2,4,6-Trichlorphenyl | |
| 243 | 1 | H | H | $C_2H_5$ | 2-Chlor-3-fluorphenyl | |
| 244 | 1 | H | H | $C_2H_5$ | 2-Chlor-4-fluorphenyl | |
| 245 | 1 | H | H | $C_2H_5$ | 2-Chlor-4-fluorphenyl | |
| 246 | 1 | H | H | $C_2H_5$ | 3-Chlor-4-methoxyphenyl | |
| 247 | 1 | H | H | $C_2H_5$ | 2-Chlor-4-methylphenyl | |
| 248 | 1 | H | H | $C_2H_5$ | 3-Chlor-4-hydroxyphenyl | |
| 249 | 1 | H | H | $C_2H_5$ | 2-Chlor-6-fluorphenyl | |
| 250 | 1 | H | H | $C_2H_5$ | 3-Fluor-4-methoxyphenyl | |
| 251 | 1 | H | H | $C_2H_5$ | 2-Bromphenyl | |
| 252 | 1 | H | H | $C_2H_5$ | 3-Bromphenyl | |
| 253 | 1 | H | H | $C_2H_5$ | 4-Bromphenyl | |
| 254 | 1 | H | H | $C_2H_5$ | 2-Brom-4-fluorphenyl | |
| 255 | 1 | H | H | $C_2H_5$ | 2-Brom-4-chlorphenyl | |
| 256 | 1 | H | H | $C_2H_5$ | 2-Methoxyphenyl | |

Fortsetzung Tabelle

| Nr. | n | $R^1$ | $R^2$ | $R^3$ | Ar | physik. Daten IR ($cm^{-1}$) |
|---|---|---|---|---|---|---|
| 257 | 1 | H | H | $C_2H_5$ | 3-Methoxyphenyl | |
| 258 | 1 | H | H | $C_2H_5$ | 4-Methoxyphenyl | |
| 259 | 1 | H | H | $C_2H_5$ | 4-Butoxyphenyl | |
| 260 | 1 | H | H | $C_2H_5$ | 4-n-Propoxyphenyl | |
| 261 | 1 | H | H | $C_2H_5$ | 4-tert-.Butoxyphenyl | |
| 262 | 1 | H | H | $C_2H_5$ | 2-Trifluormethylphenyl | |
| 263 | 1 | H | H | $C_2H_5$ | 3-Trifluormethylphenyl | |
| 264 | 1 | H | H | $C_2H_5$ | 4-Trifluormethylphenyl | |
| 265 | 1 | H | H | $C_2H_5$ | 4-Trifluormethoxyphenyl | |
| 266 | 1 | H | H | $C_2H_5$ | 4-Tetrafluorethoxyphenyl | |
| 267 | 1 | H | H | $C_2H_5$ | 3,4-Dimethoxyphenyl | |
| 268 | 1 | H | H | $C_2H_5$ | 2-Methoxy-4-trifluormethylphenyl | |
| 269 | 1 | H | H | $C_2H_5$ | 4-Phenoxyphenyl | |
| 270 | 1 | H | H | $C_2H_5$ | 4-(4'-Chlorphenoxy)-phenyl | |
| 271 | 1 | H | H | $C_2H_5$ | 2-Nitrophenyl | |
| 272 | 1 | H | H | $C_2H_5$ | 4-Nitrophenyl | |
| 273 | 1 | H | H | $C_2H_5$ | 2-Chlor-4-nitrophenyl | |
| 274 | 1 | H | H | $C_2H_5$ | 4-Hydroxyphenyl | |
| 275 | 1 | H | H | $C_2H_5$ | 2-Hydroxyphenyl | |
| 276 | 1 | H | H | iso-$C_3H_7$ | Phenyl | |
| 277 | 1 | H | H | iso-$C_3H_7$ | 2-Methylphenyl | |
| 278 | 1 | H | H | iso-$C_3H_7$ | 3-Methylphenyl | |
| 279 | 1 | H | H | iso-$C_3H_7$ | 4-Methylphenyl | |
| 280 | 1 | H | H | iso-$C_3H_7$ | 2,4-Dimethylphenyl | |

Fortsetzung Tabelle

| Nr. | n | $R^1$ | $R^2$ | $R^3$ | Ar | physik. Daten IR ($cm^{-1}$) |
|---|---|---|---|---|---|---|
| 281 | 1 | H | H | iso-$C_3H_7$ | 2,6-Dimethylphenyl | |
| 282 | 1 | H | H | iso-$C_3H_7$ | 2,4,6-Trimethylphenyl | |
| 283 | 1 | H | H | iso-$C_3H_7$ | 4-tert.-Butylphenyl | |
| 284 | 1 | H | H | iso-$C_3H_7$ | 1-Naphthyl | |
| 285 | 1 | H | H | iso-$C_3H_7$ | 2-Naphthyl | |
| 286 | 1 | H | H | iso-$C_3H_7$ | 4-Biphenyl | |
| 287 | 1 | H | H | iso-$C_3H_7$ | 2-Fluorphenyl | |
| 288 | 1 | H | H | iso-$C_3H_7$ | 3-Fluorphenyl | |
| 289 | 1 | H | H | iso-$C_3H_7$ | 4-Fluorphenyl | |
| 290 | 1 | H | H | iso-$C_3H_7$ | 2,4-Difluorphenyl | |
| 291 | 1 | H | H | iso-$C_3H_7$ | 2-Chlorphenyl | |
| 292 | 1 | H | H | iso-$C_3H_7$ | 3-Chlorphenyl | |
| 293 | 1 | H | H | iso-$C_3H_7$ | 4-Chlorphenyl | |
| 294 | 1 | H | H | iso-$C_3H_7$ | 2,4-Dichlorphenyl | |
| 295 | 1 | H | H | iso-$C_3H_7$ | 3,4-Dichlorphenyl | |
| 296 | 1 | H | H | iso-$C_3H_7$ | 2,6-Dichlorphenyl | |
| 297 | 1 | H | H | iso-$C_3H_7$ | 2,4,6-Trichlorphenyl | |
| 298 | 1 | H | H | iso-$C_3H_7$ | 2-Chlor-3-fluorphenyl | |
| 299 | 1 | H | H | iso-$C_3H_7$ | 2-Chlor-4-fluorphenyl | |
| 355 | 1 | $CH_3$ | $CH_3$ | $CH_3$ | 2-Chlor-4-fluorphenyl | |
| 356 | 1 | $CH_3$ | $CH_3$ | $CH_3$ | 3-Chlor-4-methoxyphenyl | |
| 357 | 1 | $CH_3$ | $CH_3$ | $CH_3$ | 2-Chlor-4-methylphenyl | |
| 358 | 1 | $CH_3$ | $CH_3$ | $CH_3$ | 3-Chlor-4-hydroxyphenyl | |
| 359 | 1 | $CH_3$ | $CH_3$ | $CH_3$ | 2-Chlor-6-fluorphenyl | |

Fortsetzung Tabelle

| Nr. | n | $R^1$ | $R^2$ | $R^3$ | Ar | physik. Daten IR ($cm^{-1}$) |
|---|---|---|---|---|---|---|
| 360 | 1 | $CH_3$ | $CH_3$ | $CH_3$ | 3-Fluor-4-methoxyphenyl | |
| 361 | 1 | $CH_3$ | $CH_3$ | $CH_3$ | 2-Bromphenyl | |
| 362 | 1 | $CH_3$ | $CH_3$ | $CH_3$ | 3-Bromphenyl | |
| 363 | 1 | $CH_3$ | $CH_3$ | $CH_3$ | 4-Bromphenyl | |
| 364 | 1 | $CH_3$ | $CH_3$ | $CH_3$ | 2-Brom-4-fluorphenyl | |
| 365 | 1 | $CH_3$ | $CH_3$ | $CH_3$ | 2-Brom-4-chlorphenyl | |
| 366 | 1 | $CH_3$ | $CH_3$ | $CH_3$ | 2-Methoxyphenyl | |
| 367 | 1 | $CH_3$ | $CH_3$ | $CH_3$ | 3-Methoxyphenyl | |
| 368 | 1 | $CH_3$ | $CH_3$ | $CH_3$ | 4-Methoxyphenyl | |
| 369 | 1 | $CH_3$ | $CH_3$ | $CH_3$ | 4-Butoxyphenyl | |
| 370 | 1 | $CH_3$ | $CH_3$ | $CH_3$ | 4-n-Propoxyphenyl | |
| 371 | 1 | $CH_3$ | $CH_3$ | $CH_3$ | 4-tert-.Butoxyphenyl | |
| 372 | 1 | $CH_3$ | $CH_3$ | $CH_3$ | 2-Trifluormethylphenyl | |
| 373 | 1 | $CH_3$ | $CH_3$ | $CH_3$ | 3-Trifluormethylphenyl | |
| 374 | 1 | $CH_3$ | $CH_3$ | $CH_3$ | 4-Trifluormethylphenyl | |
| 375 | 1 | $CH_3$ | $CH_3$ | $CH_3$ | 4-Trifluormethoxyphenyl | |
| 376 | 1 | $CH_3$ | $CH_3$ | $CH_3$ | 4-Tetrafluorethoxyphenyl | |
| 377 | 1 | $CH_3$ | $CH_3$ | $CH_3$ | 3,4-Dimethoxyphenyl | |
| 378 | 1 | $CH_3$ | $CH_3$ | $CH_3$ | 2-Methoxy-4-trifluormethylphenyl | |
| 379 | 1 | $CH_3$ | $CH_3$ | $CH_3$ | 4-Phenoxyphenyl | |
| 380 | 1 | $CH_3$ | $CH_3$ | $CH_3$ | 4-(4'-Chlorphenoxy)-phenyl | |
| 381 | 1 | $CH_3$ | $CH_3$ | $CH_3$ | 2-Nitrophenyl | |
| 382 | 1 | $CH_3$ | $CH_3$ | $CH_3$ | 4-Nitrophenyl | |
| 383 | 1 | $CH_3$ | $CH_3$ | $CH_3$ | 2-Chlor-4-nitrophenyl | |

Fortsetzung Tabelle

| Nr. | n | $R^1$ | $R^2$ | $R^3$ | Ar | physik. Daten IR ($cm^{-1}$) |
|---|---|---|---|---|---|---|
| 384 | 1 | $CH_3$ | $CH_3$ | $CH_3$ | 4-Hydroxyphenyl | |
| 385 | 1 | $CH_3$ | $CH_3$ | $CH_3$ | 2-Hydroxyphenyl | |
| 386 | 1 | $CH_3$ | $CH_3$ | $C_2H_5$ | Phenyl | |
| 387 | 1 | $CH_3$ | $CH_3$ | $C_2H_5$ | 2-Methylphenyl | |
| 388 | 1 | $CH_3$ | $CH_3$ | $C_2H_5$ | 3-Methylphenyl | |
| 389 | 1 | $CH_3$ | $CH_3$ | $C_2H_5$ | 4-Methylphenyl | |
| 390 | 1 | $CH_3$ | $CH_3$ | $C_2H_5$ | 2,4-Dimethylphenyl | |
| 391 | 1 | $CH_3$ | $CH_3$ | $C_2H_5$ | 2,6-Dimethylphenyl | |
| 392 | 1 | $CH_3$ | $CH_3$ | $C_2H_5$ | 2,4,6-Trimethylphenyl | |
| 393 | 1 | $CH_3$ | $CH_3$ | $C_2H_5$ | 4-tert.-Butylphenyl | |
| 394 | 1 | $CH_3$ | $CH_3$ | $C_2H_5$ | 1-Naphthyl | |
| 395 | 1 | $CH_3$ | $CH_3$ | $C_2H_5$ | 2-Naphthyl | |
| 396 | 1 | $CH_3$ | $CH_3$ | $C_2H_5$ | 4-Biphenyl | |
| 397 | 1 | $CH_3$ | $CH_3$ | $C_2H_5$ | 2-Fluorphenyl | |
| 398 | 1 | $CH_3$ | $CH_3$ | $C_2H_5$ | 3-Fluorphenyl | |
| 399 | 1 | $CH_3$ | $CH_3$ | $C_2H_5$ | 4-Fluorphenyl | |
| 400 | 1 | $CH_3$ | $CH_3$ | $C_2H_5$ | 2,4-Difluorphenyl | |
| 401 | 1 | $CH_3$ | $CH_3$ | $C_2H_5$ | 2-Chlorphenyl | |
| 402 | 1 | $CH_3$ | $CH_3$ | $C_2H_5$ | 3-Chlorphenyl | |
| 403 | 1 | $CH_3$ | $CH_3$ | $C_2H_5$ | 4-Chlorphenyl | |
| 404 | 1 | $CH_3$ | $CH_3$ | $C_2H_5$ | 2,4-Dichlorphenyl | |
| 405 | 1 | $CH_3$ | $CH_3$ | $C_2H_5$ | 3,4-Dichlorphenyl | |
| 406 | 1 | $CH_3$ | $CH_3$ | $C_2H_5$ | 2,6-Dichlorphenyl | |
| 407 | 1 | $CH_3$ | $CH_3$ | $C_2H_5$ | 2,4,6-Trichlorphenyl | |

Fortsetzung Tabelle

| Nr. | n | $R^1$ | $R^2$ | $R^3$ | Ar | physik. Daten IR ($cm^{-1}$) |
|---|---|---|---|---|---|---|
| 408 | 1 | $CH_3$ | $CH_3$ | $C_2H_5$ | 2-Chlor-3-fluorphenyl | |
| 409 | 1 | $CH_3$ | $CH_3$ | $C_2H_5$ | 2-Chlor-4-fluorphenyl | |
| 410 | 1 | $CH_3$ | $CH_3$ | $C_2H_5$ | 2-Chlor-4-fluorphenyl | |
| 411 | 1 | $CH_3$ | $CH_3$ | $C_2H_5$ | 3-Chlor-4-methoxyphenyl | |
| 412 | 1 | $CH_3$ | $CH_3$ | $C_2H_5$ | 2-Chlor-4-methylphenyl | |
| 413 | 1 | $CH_3$ | $CH_3$ | $C_2H_5$ | 3-Chlor-4-hydroxyphenyl | |
| 414 | 1 | $CH_3$ | $CH_3$ | $C_2H_5$ | 2-Chlor-6-fluorphenyl | |
| 415 | 1 | $CH_3$ | $CH_3$ | $C_2H_5$ | 3-Fluor-4-methoxyphenyl | |
| 416 | 1 | $CH_3$ | $CH_3$ | $C_2H_5$ | 2-Bromphenyl | |
| 417 | 1 | $CH_3$ | $CH_3$ | $C_2H_5$ | 3-Bromphenyl | |
| 418 | 1 | $CH_3$ | $CH_3$ | $C_2H_5$ | 4-Bromphenyl | |
| 419 | 1 | $CH_3$ | $CH_3$ | $C_2H_5$ | 2-Brom-4-fluorphenyl | |
| 420 | 1 | $CH_3$ | $CH_3$ | $C_2H_5$ | 2-Brom-4-chlorphenyl | |
| 421 | 1 | $CH_3$ | $CH_3$ | $C_2H_5$ | 2-Methoxyphenyl | |
| 422 | 1 | $CH_3$ | $CH_3$ | $C_2H_5$ | 3-Methoxyphenyl | |
| 423 | 1 | $CH_3$ | $CH_3$ | $C_2H_5$ | 4-Methoxyphenyl | |
| 424 | 1 | $CH_3$ | $CH_3$ | $C_2H_5$ | 4-Butoxyphenyl | |
| 425 | 1 | $CH_3$ | $CH_3$ | $C_2H_5$ | 4-n-Propoxyphenyl | |
| 426 | 1 | $CH_3$ | $CH_3$ | $C_2H_5$ | 4-tert-.Butoxyphenyl | |
| 427 | 1 | $CH_3$ | $CH_3$ | $C_2H_5$ | 2-Trifluormethylphenyl | |
| 428 | 1 | $CH_3$ | $CH_3$ | $C_2H_5$ | 3-Trifluormethylphenyl | |
| 429 | 1 | $CH_3$ | $CH_3$ | $C_2H_5$ | 4-Trifluormethylphenyl | |
| 430 | 1 | $CH_3$ | $CH_3$ | $C_2H_5$ | 4-Trifluormethoxyphenyl | |
| 431 | 1 | $CH_3$ | $CH_3$ | $C_2H_5$ | 4-Tetrafluorethoxyphenyl | |

Fortsetzung Tabelle

| Nr. | n | $R^1$ | $R^2$ | $R^3$ | Ar | physik. Daten IR ($cm^{-1}$) |
|---|---|---|---|---|---|---|
| 432 | 1 | $CH_3$ | $CH_3$ | $C_2H_5$ | 3,4-Dimethoxyphenyl | |
| 433 | 1 | $CH_3$ | $CH_3$ | $C_2H_5$ | 2-Methoxy-4-trifluormethylphenyl | |
| 434 | 1 | $CH_3$ | $CH_3$ | $C_2H_5$ | 4-Phenoxyphenyl | |
| 435 | 1 | $CH_3$ | $CH_3$ | $C_2H_5$ | 4-(4'-Chlorphenoxy)-phenyl | |
| 436 | 1 | $CH_3$ | $CH_3$ | $C_2H_5$ | 2-Nitrophenyl | |
| 437 | 1 | $CH_3$ | $CH_3$ | $C_2H_5$ | 4-Nitrophenyl | |
| 438 | 1 | $CH_3$ | $CH_3$ | $C_2H_5$ | 2-Chlor-4-nitrophenyl | |
| 439 | 1 | $CH_3$ | $CH_3$ | $C_2H_5$ | 4-Hydroxyphenyl | |
| 440 | 1 | $CH_3$ | $CH_3$ | $C_2H_5$ | 2-Hydroxyphenyl | |
| 441 | 2 | H | H | $CH_3$ | Phenyl | |
| 442 | 2 | H | H | $CH_3$ | 2-Methylphenyl | |
| 443 | 2 | H | H | $CH_3$ | 3-Methylphenyl | |
| 444 | 2 | H | H | $CH_3$ | 4-Methylphenyl | |
| 445 | 2 | H | H | $CH_3$ | 2,4-Dimethylphenyl | |
| 446 | 2 | H | H | $CH_3$ | 2,6-Dimethylphenyl | |
| 447 | 2 | H | H | $CH_3$ | 2,4,6-Trimethylphenyl | |
| 448 | 2 | H | H | $CH_3$ | 4-tert.-Butylphenyl | |
| 449 | 2 | H | H | $CH_3$ | 1-Naphthyl | |
| 450 | 2 | H | H | $CH_3$ | 2-Naphthyl | |
| 451 | 2 | H | H | $CH_3$ | 4-Biphenyl | |
| 452 | 2 | H | H | $CH_3$ | 2-Fluorphenyl | |
| 453 | 2 | H | H | $CH_3$ | 3-Fluorphenyl | |
| 454 | 2 | H | H | $CH_3$ | 4-Fluorphenyl | |
| 455 | 2 | H | H | $CH_3$ | 2,4-Difluorphenyl | |

Fortsetzung Tabelle

| Nr. | n | $R^1$ | $R^2$ | $R^3$ | Ar | physik. Daten IR ($cm^{-1}$) |
|---|---|---|---|---|---|---|
| 456 | 2 | H | H | $CH_3$ | 2-Chlorphenyl | |
| 457 | 2 | H | H | $CH_3$ | 3-Chlorphenyl | |
| 458 | 2 | H | H | $CH_3$ | 4-Chlorphenyl | |
| 459 | 2 | H | H | $CH_3$ | 2,4-Dichlorphenyl | |
| 460 | 2 | H | H | $CH_3$ | 3,4-Dichlorphenyl | |
| 461 | 2 | H | H | $CH_3$ | 2,6-Dichlorphenyl | |
| 462 | 2 | H | H | $CH_3$ | 2,4,6-Trichlorphenyl | |
| 463 | 2 | H | H | $CH_3$ | 2-Chlor-3-fluorphenyl | |
| 464 | 2 | H | H | $CH_3$ | 2-Chlor-4-fluorphenyl | |
| 465 | 2 | H | H | $CH_3$ | 2-Chlor-4-fluorphenyl | |
| 466 | 2 | H | H | $CH_3$ | 3-Chlor-4-methoxyphenyl | |
| 467 | 2 | H | H | $CH_3$ | 2-Chlor-4-methylphenyl | |
| 468 | 2 | H | H | $CH_3$ | 3-Chlor-4-hydroxyphenyl | |
| 469 | 2 | H | H | $CH_3$ | 2-Chlor-6-fluorphenyl | |
| 470 | 2 | H | H | $CH_3$ | 3-Fluor-4-methoxyphenyl | |
| 471 | 2 | H | H | $CH_3$ | 2-Bromphenyl | |
| 472 | 2 | H | H | $CH_3$ | 3-Bromphenyl | |
| 473 | 2 | H | H | $CH_3$ | 4-Bromphenyl | |
| 474 | 2 | H | H | $CH_3$ | 2-Brom-4-fluorphenyl | |
| 475 | 2 | H | H | $CH_3$ | 2-Brom-4-chlorphenyl | |
| 476 | 2 | H | H | $CH_3$ | 2-Methoxyphenyl | |
| 477 | 2 | H | H | $CH_3$ | 3-Methoxyphenyl | |
| 478 | 2 | H | H | $CH_3$ | 4-Methoxyphenyl | |
| 479 | 2 | H | H | $CH_3$ | 4-Butoxyphenyl | |

Fortsetzung Tabelle

| Nr. | n | $R^1$ | $R^2$ | $R^3$ | Ar | physik. Daten IR ($cm^{-1}$) |
|---|---|---|---|---|---|---|
| 480 | 2 | H | H | $CH_3$ | 4-n-Propoxyphenyl | |
| 481 | 2 | H | H | $CH_3$ | 4-tert-.Butoxyphenyl | |
| 482 | 2 | H | H | $CH_3$ | 2-Trifluormethylphenyl | |
| 483 | 2 | H | H | $CH_3$ | 3-Trifluormethylphenyl | |
| 484 | 2 | H | H | $CH_3$ | 4-Trifluormethylphenyl | |
| 485 | 2 | H | H | $CH_3$ | 4-Trifluormethoxyphenyl | |
| 465 | 2 | H | H | $CH_3$ | 4-Tetrafluorethoxyphenyl | |
| 466 | 2 | H | H | $CH_3$ | 3,4-Dimethoxyphenyl | |
| 467 | 2 | H | H | $CH_3$ | 2-Methoxy-4-trifluormethylphenyl | |
| 468 | 2 | H | H | $CH_3$ | 4-Phenoxyphenyl | |
| 469 | 2 | H | H | $CH_3$ | 4-(4'-Chlorphenoxy)-phenyl | |
| 470 | 2 | H | H | $CH_3$ | 2-Nitrophenyl | |
| 471 | 2 | H | H | $CH_3$ | 4-Nitrophenyl | |
| 472 | 2 | H | H | $CH_3$ | 2-Chlor-4-nitrophenyl | |
| 473 | 2 | H | H | $CH_3$ | 4-Hydroxyphenyl | |
| 474 | 2 | H | H | $CH_3$ | 2-Hydroxyphenyl | |
| 475 | 0 | - | - | n-$C_4H_9$ | Phenyl | |
| 476 | 0 | - | - | n-$C_4H_9$ | 2-Methylphenyl | |
| 477 | 0 | - | - | n-$C_4H_9$ | 4-Methylphenyl | |
| 478 | 0 | - | - | n-$C_4H_9$ | 2,4-Dimethylphenyl | |
| 479 | 0 | - | - | n-$C_4H_9$ | 2,4,6-Trimethylphenyl | |
| 480 | 0 | - | - | n-$C_4H_9$ | 4-tert.-Butylphenyl | |
| 481 | 0 | - | - | n-$C_4H_9$ | 2-Naphthyl | |
| 482 | 0 | - | - | n-$C_4H_9$ | 4-Biphenyl | |

Fortsetzung Tabelle

| Nr. | n | $R^1$ | $R^2$ | $R^3$ | Ar | physik. Daten IR ($cm^{-1}$) |
|---|---|---|---|---|---|---|
| 483 | 0 | – | – | n-$C_4H_9$ | 2-Fluorphenyl | |
| 484 | 0 | – | – | n-$C_4H_9$ | 4-Fluorphenyl | |
| 485 | 0 | – | – | n-$C_4H_9$ | 2,4-Difluorphenyl | |
| 486 | 0 | – | – | n-$C_4H_9$ | 2-Chlorphenyl | |
| 487 | 0 | – | – | n-$C_4H_9$ | 4-Chlorphenyl | |
| 488 | 0 | – | – | n-$C_4H_9$ | 2,4-Dichlorphenyl | |
| 489 | 0 | – | – | n-$C_4H_9$ | 2-Chlor-4-fluorphenyl | |
| 490 | 0 | – | – | n-$C_4H_9$ | 3-Chlor-4-hydroxyphenyl | |
| 491 | 0 | – | – | n-$C_4H_9$ | 2-Methoxyphenyl | |
| 492 | 0 | – | – | n-$C_4H_9$ | 3-Chlor-4-methoxyphenyl | |
| 493 | 0 | – | – | n-$C_4H_9$ | 4-Methoxyphenyl | |
| 494 | 0 | – | – | n-$C_4H_9$ | 2-Trifluormethylphenyl | |
| 495 | 0 | – | – | n-$C_4H_9$ | 4-Trifluormethylphenyl | |
| 496 | 0 | – | – | n-$C_4H_9$ | 4-Trifluormethoxyphenyl | |
| 497 | 0 | – | – | n-$C_4H_9$ | 4-Hydroxyphenyl | |
| 498 | 0 | – | – | n-$C_4H_9$ | 2-Hydroxyphenyl | |
| 499 | 1 | H | H | n-$C_4H_9$ | Phenyl | |
| 500 | 1 | H | H | n-$C_4H_9$ | 2-Methylphenyl | |
| 501 | 1 | H | H | n-$C_4H_9$ | 4-Methylphenyl | |
| 502 | 1 | H | H | n-$C_4H_9$ | 2,4-Dimethylphenyl | |
| 503 | 1 | H | H | n-$C_4H_9$ | 2,4,6-Trimethylphenyl | |
| 504 | 1 | H | H | n-$C_4H_9$ | 4-tert.-Butylphenyl | |
| 505 | 1 | H | H | n-$C_4H_9$ | 2-Naphthyl | |
| 506 | 1 | H | H | n-$C_4H_9$ | 4-Biphenyl | |

Fortsetzung Tabelle

| Nr. | n | $R^1$ | $R^2$ | $R^3$ | Ar | physik. Daten IR ($cm^{-1}$) |
|---|---|---|---|---|---|---|
| 507 | 1 | H | H | n-$C_4H_9$ | 2-Fluorphenyl | |
| 508 | 1 | H | H | n-$C_4H_9$ | 4-Fluorphenyl | |
| 509 | 1 | H | H | n-$C_4H_9$ | 2,4-Difluorphenyl | |
| 510 | 1 | H | H | n-$C_4H_9$ | 2-Chlorphenyl | |
| 511 | 1 | H | H | n-$C_4H_9$ | 4-Chlorphenyl | |
| 512 | 1 | H | H | n-$C_4H_9$ | 2,4-Dichlorphenyl | |
| 513 | 1 | H | H | n-$C_4H_9$ | 2-Chlor-4-fluorphenyl | |
| 514 | 1 | H | H | n-$C_4H_9$ | 3-Chlor-4-hydroxyphenyl | |
| 515 | 1 | H | H | n-$C_4H_9$ | 2-Methoxyphenyl | |
| 516 | 1 | H | H | n-$C_4H_9$ | 3-Chlor-4-methoxyphenyl | |
| 517 | 1 | H | H | n-$C_4H_9$ | 4-Methoxyphenyl | |
| 518 | 1 | H | H | n-$C_4H_9$ | 2-Trifluormethylphenyl | |
| 519 | 1 | H | H | n-$C_4H_9$ | 4-Trifluormethylphenyl | |
| 520 | 1 | H | H | n-$C_4H_9$ | 4-Trifluormethoxyphenyl | |
| 521 | 1 | H | H | n-$C_4H_9$ | 4-Hydroxyphenyl | |
| 522 | 1 | H | H | n-$C_4H_9$ | 2-Hydroxyphenyl | |
| 523 | 1 | $CH_3$ | $CH_3$ | n-$C_4H_9$ | Phenyl | |
| 524 | 1 | $CH_3$ | $CH_3$ | n-$C_4H_9$ | 2-Methylphenyl | |
| 525 | 1 | $CH_3$ | $CH_3$ | n-$C_4H_9$ | 4-Methylphenyl | |
| 526 | 1 | $CH_3$ | $CH_3$ | n-$C_4H_9$ | 2,4-Dimethylphenyl | |
| 527 | 1 | $CH_3$ | $CH_3$ | n-$C_4H_9$ | 2,4,6-Trimethylphenyl | |
| 528 | 1 | $CH_3$ | $CH_3$ | n-$C_4H_9$ | 4-tert.-Butylphenyl | |
| 529 | 1 | $CH_3$ | $CH_3$ | n-$C_4H_9$ | 2-Naphthyl | |
| 530 | 1 | $CH_3$ | $CH_3$ | n-$C_4H_9$ | 4-Biphenyl | |

Fortsetzung Tabelle

| Nr. | n | $R^1$ | $R^2$ | $R^3$ | Ar | physik. Daten IR ($cm^{-1}$) |
|---|---|---|---|---|---|---|
| 531 | 1 | $CH_3$ | $CH_3$ | n-$C_4H_9$ | 2-Fluorphenyl | |
| 532 | 1 | $CH_3$ | $CH_3$ | n-$C_4H_9$ | 4-Fluorphenyl | |
| 533 | 1 | $CH_3$ | $CH_3$ | n-$C_4H_9$ | 2,4-Difluorphenyl | |
| 534 | 1 | $CH_3$ | $CH_3$ | n-$C_4H_9$ | 2-Chlorphenyl | |
| 535 | 1 | $CH_3$ | $CH_3$ | n-$C_4H_9$ | 4-Chlorphenyl | |
| 536 | 1 | $CH_3$ | $CH_3$ | n-$C_4H_9$ | 2,4-Dichlorphenyl | |
| 537 | 1 | $CH_3$ | $CH_3$ | n-$C_4H_9$ | 2-Chlor-4-fluorphenyl | |
| 538 | 1 | $CH_3$ | $CH_3$ | n-$C_4H_9$ | 3-Chlor-4-hydroxyphenyl | |
| 539 | 1 | $CH_3$ | $CH_3$ | n-$C_4H_9$ | 2-Methoxyphenyl | |
| 540 | 1 | $CH_3$ | $CH_3$ | n-$C_4H_9$ | 3-Chlor-4-methoxyphenyl | |
| 541 | 1 | $CH_3$ | $CH_3$ | n-$C_4H_9$ | 4-Methoxyphenyl | |
| 542 | 1 | $CH_3$ | $CH_3$ | n-$C_4H_9$ | 2-Trifluormethylphenyl | |
| 543 | 1 | $CH_3$ | $CH_3$ | n-$C_4H_9$ | 4-Trifluormethylphenyl | |
| 544 | 1 | $CH_3$ | $CH_3$ | n-$C_4H_9$ | 4-Trifluormethoxyphenyl | |
| 545 | 1 | $CH_3$ | $CH_3$ | n-$C_4H_9$ | 4-Hydroxyphenyl | |
| 546 | 1 | $CH_3$ | $CH_3$ | n-$C_4H_9$ | 2-Hydroxyphenyl | |
| 547 | 0 | - | - | $CH_2$-$C_6H_5$ | Phenyl | |
| 548 | 0 | - | - | $CH_2$-$C_6H_5$ | 2-Methylphenyl | |
| 549 | 0 | - | - | $CH_2$-$C_6H_5$ | 4-Methylphenyl | |
| 550 | 0 | - | - | $CH_2$-$C_6H_5$ | 2,4-Dimethylphenyl | |
| 551 | 0 | - | - | $CH_2$-$C_6H_5$ | 2,4,6-Trimethylphenyl | |
| 552 | 0 | - | - | $CH_2$-$C_6H_5$ | 4-tert.-Butylphenyl | |
| 553 | 0 | - | - | $CH_2$-$C_6H_5$ | 2-Naphthyl | |
| 554 | 0 | - | - | $CH_2$-$C_6H_5$ | 4-Biphenyl | |

Fortsetzung Tabelle

| Nr. | n | $R^1$ | $R^2$ | $R^3$ | Ar | physik. Daten IR ($cm^{-1}$) |
|---|---|---|---|---|---|---|
| 555 | 0 | - | - | $CH_2-C_6H_5$ | 2-Fluorphenyl | |
| 556 | 0 | - | - | $CH_2-C_6H_5$ | 4-Fluorphenyl | |
| 557 | 0 | - | - | $CH_2-C_6H_5$ | 2,4-Difluorphenyl | |
| 558 | 0 | - | - | $CH_2-C_6H_5$ | 2-Chlorphenyl | |
| 559 | 0 | - | - | $CH_2-C_6H_5$ | 4-Chlorphenyl | |
| 560 | 0 | - | - | $CH_2-C_6H_5$ | 2,4-Dichlorphenyl | |
| 561 | 0 | - | - | $CH_2-C_6H_5$ | 2-Chlor-4-fluorphenyl | |
| 562 | 0 | - | - | $CH_2-C_6H_5$ | 3-Chlor-4-hydroxyphenyl | |
| 563 | 0 | - | - | $CH_2-C_6H_5$ | 2-Methoxyphenyl | |
| 564 | 0 | - | - | $CH_2-C_6H_5$ | 3-Chlor-4-methoxyphenyl | |
| 565 | 0 | - | - | $CH_2-C_6H_5$ | 4-Methoxyphenyl | |
| 566 | 0 | - | - | $CH_2-C_6H_5$ | 2-Trifluormethylphenyl | |
| 567 | 0 | - | - | $CH_2-C_6H_5$ | 4-Trifluormethylphenyl | |
| 568 | 0 | - | - | $CH_2-C_6H_5$ | 4-Trifluormethoxyphenyl | |
| 569 | 0 | - | - | $CH_2-C_6H_5$ | 4-Hydroxyphenyl | |
| 570 | 0 | - | - | $CH_2-C_6H_5$ | 2-Hydroxyphenyl | |
| 571 | 1 | H | H | $CH_2-C_6H_5$ | Phenyl | |
| 572 | 1 | H | H | $CH_2-C_6H_5$ | 2-Methylphenyl | |
| 573 | 1 | H | H | $CH_2-C_6H_5$ | 4-Methylphenyl | |
| 574 | 1 | H | H | $CH_2-C_6H_5$ | 2,4-Dimethylphenyl | |
| 575 | 1 | H | H | $CH_2-C_6H_5$ | 2,4,6-Trimethylphenyl | |
| 576 | 1 | H | H | $CH_2-C_6H_5$ | 4-tert.-Butylphenyl | |
| 577 | 1 | H | H | $CH_2-C_6H_5$ | 2-Naphthyl | |
| 578 | 1 | H | H | $CH_2-C_6H_5$ | 4-Biphenyl | |

Fortsetzung Tabelle

| Nr. | n | $R^1$ | $R^2$ | $R^3$ | Ar | physik. Daten IR ($cm^{-1}$) |
|---|---|---|---|---|---|---|
| 579 | 1 | H | H | $CH_2-C_6H_5$ | 2-Fluorphenyl | |
| 580 | 1 | H | H | $CH_2-C_6H_5$ | 4-Fluorphenyl | |
| 581 | 1 | H | H | $CH_2-C_6H_5$ | 2,4-Difluorphenyl | |
| 582 | 1 | H | H | $CH_2-C_6H_5$ | 2-Chlorphenyl | |
| 583 | 1 | H | H | $CH_2-C_6H_5$ | 4-Chlorphenyl | |
| 584 | 1 | H | H | $CH_2-C_6H_5$ | 2,4-Dichlorphenyl | |
| 585 | 1 | H | H | $CH_2-C_6H_5$ | 2-Chlor-4-fluorphenyl | |
| 586 | 1 | H | H | $CH_2-C_6H_5$ | 3-Chlor-4-hydroxyphenyl | |
| 587 | 1 | H | H | $CH_2-C_6H_5$ | 2-Methoxyphenyl | |
| 588 | 1 | H | H | $CH_2-C_6H_5$ | 3-Chlor-4-methoxyphenyl | |
| 589 | 1 | H | H | $CH_2-C_6H_5$ | 4-Methoxyphenyl | |
| 590 | 1 | H | H | $CH_2-C_6H_5$ | 2-Trifluormethylphenyl | |
| 591 | 1 | H | H | $CH_2-C_6H_5$ | 4-Trifluormethylphenyl | |
| 592 | 1 | H | H | $CH_2-C_6H_5$ | 4-Trifluormethoxyphenyl | |
| 593 | 1 | H | H | $CH_2-C_6H_5$ | 4-Hydroxyphenyl | |
| 594 | 1 | H | H | $CH_2-C_6H_5$ | 2-Hydroxyphenyl | |

Fortsetzung Tabelle

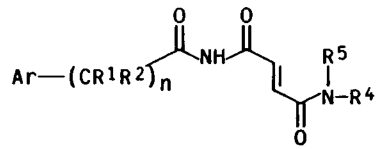

| Nr. | n | $R^1$ | $R^2$ | $R^4$ | $R^5$ | Ar | physik. Daten IR ($cm^{-1}$) |
|---|---|---|---|---|---|---|---|
| 595 | 0 | - | - | H | H | Phenyl | |
| 596 | 0 | - | - | H | H | 2-Methylphenyl | |
| 597 | 0 | - | - | H | H | 3-Methylphenyl | |
| 598 | 0 | - | - | H | H | 4-Methylphenyl | |
| 599 | 0 | - | - | H | H | 2,4-Dimethylphenyl | |
| 600 | 0 | - | - | H | H | 2,6-Dimethylphenyl | |
| 601 | 0 | - | - | H | H | 2,4,6-Trimethylphenyl | |
| 602 | 0 | - | - | H | H | 4-tert.-Butylphenyl | |
| 603 | 0 | - | - | H | H | 1-Naphthyl | |
| 604 | 0 | - | - | H | H | 2-Naphthyl | |
| 605 | 0 | - | - | H | H | 4-Biphenyl | |
| 606 | 0 | - | - | H | H | 2-Fluorphenyl | |
| 607 | 0 | - | - | H | H | 3-Fluorphenyl | |
| 608 | 0 | - | - | H | H | 4-Fluorphenyl | |
| 609 | 0 | - | - | H | H | 2,4-Difluorphenyl | |
| 610 | 0 | - | - | H | H | 2-Chlorphenyl | |
| 611 | 0 | - | - | H | H | 3-Chlorphenyl | |
| 612 | 0 | - | - | H | H | 4-Chlorphenyl | |
| 613 | 0 | - | - | H | H | 2,4-Dichlorphenyl | |

Fortsetzung Tabelle

| Nr. | n | $R^1$ | $R^2$ | $R^4$ | $R^5$ | Ar | physik. Daten IR ($cm^{-1}$) |
|---|---|---|---|---|---|---|---|
| 614 | 0 | - | - | H | H | 3,4-Dichlorphenyl | |
| 615 | 0 | - | - | H | H | 2,6-Dichlorphenyl | |
| 616 | 0 | - | - | H | H | 2,4,6-Trichlorphenyl | |
| 617 | 0 | - | - | H | H | 2-Chlor-3-fluorphenyl | |
| 618 | 0 | - | - | H | H | 2-Chlor-4-fluorphenyl | |
| 619 | 0 | - | - | H | H | 2-Chlor-4-fluorphenyl | |
| 620 | 0 | - | - | H | H | 3-Chlor-4-methoxyphenyl | |
| 621 | 0 | - | - | H | H | 2-Chlor-4-methylphenyl | |
| 622 | 0 | - | - | H | H | 3-Chlor-4-hydroxyphenyl | |
| 623 | 0 | - | - | H | H | 2-Chlor-6-fluorphenyl | |
| 624 | 0 | - | - | H | H | 3-Fluor-4-methoxyphenyl | |
| 625 | 0 | - | - | H | H | 2-Bromphenyl | |
| 626 | 0 | - | - | H | H | 3-Bromphenyl | |
| 627 | 0 | - | - | H | H | 4-Bromphenyl | |
| 628 | 0 | - | - | H | H | 2-Brom-4-fluorphenyl | |
| 629 | 0 | - | - | H | H | 2-Brom-4-chlorphenyl | |
| 630 | 0 | - | - | H | H | 2-Methoxyphenyl | |
| 631 | 0 | - | - | H | H | 3-Methoxyphenyl | |
| 632 | 0 | - | - | H | H | 4-Methoxyphenyl | |
| 633 | 0 | - | - | H | H | 4-Butoxyphenyl | |
| 634 | 0 | - | - | H | H | 4-n-Propoxyphenyl | |
| 635 | 0 | - | - | H | H | 4-tert-.Butoxyphenyl | |
| 636 | 0 | - | - | H | H | 2-Trifluormethylphenyl | |
| 637 | 0 | - | - | H | H | 3-Trifluormethylphenyl | |

Fortsetzung Tabelle

| Nr. | n | $R^1$ | $R^2$ | $R^4$ | $R^5$ | Ar | physik. Daten IR ($cm^{-1}$) |
|---|---|---|---|---|---|---|---|
| 638 | 0 | - | - | H | H | 4-Trifluormethylphenyl | |
| 639 | 0 | - | - | H | H | 4-Trifluormethoxyphenyl | |
| 640 | 0 | - | - | H | H | 4-Tetrafluorethoxyphenyl | |
| 641 | 0 | - | - | H | H | 3,4-Dimethoxyphenyl | |
| 642 | 0 | - | - | H | H | 2-Methoxy-4-trifluormethylphenyl | |
| 643 | 0 | - | - | H | H | 4-Phenoxyphenyl | |
| 644 | 0 | - | - | H | H | 4-(4'-Chlorphenoxy)-phenyl | |
| 645 | 0 | - | - | H | H | 2-Nitrophenyl | |
| 646 | 0 | - | - | H | H | 4-Nitrophenyl | |
| 647 | 0 | - | - | H | H | 2-Chlor-4-nitrophenyl | |
| 648 | 0 | - | - | H | H | 4-Hydroxyphenyl | |
| 649 | 0 | - | - | H | H | 2-Hydroxyphenyl | |
| 650 | 0 | - | - | $CH_3$ | H | Phenyl | |
| 651 | 0 | - | - | $CH_3$ | H | 2-Methylphenyl | |
| 652 | 0 | - | - | $CH_3$ | H | 3-Methylphenyl | |
| 653 | 0 | - | - | $CH_3$ | H | 4-Methylphenyl | |
| 654 | 0 | - | - | $CH_3$ | H | 2,4-Dimethylphenyl | |
| 655 | 0 | - | - | $CH_3$ | H | 2,6-Dimethylphenyl | |
| 656 | 0 | - | - | $CH_3$ | H | 2,4,6-Trimethylphenyl | |
| 657 | 0 | - | - | $CH_3$ | H | 4-tert.-Butylphenyl | |
| 658 | 0 | - | - | $CH_3$ | H | 1-Naphthyl | |
| 659 | 0 | - | - | $CH_3$ | H | 2-Naphthyl | |
| 660 | 0 | - | - | $CH_3$ | H | 4-Biphenyl | |
| 661 | 0 | - | - | $CH_3$ | H | 2-Fluorphenyl | |

Fortsetzung Tabelle

| Nr. | n | $R^1$ | $R^2$ | $R^4$ | $R^5$ | Ar | physik. Daten IR ($cm^{-1}$) |
|---|---|---|---|---|---|---|---|
| 662 | 0 | - | - | $CH_3$ | H | 3-Fluorphenyl | |
| 663 | 0 | - | - | $CH_3$ | H | 4-Fluorphenyl | |
| 664 | 0 | - | - | $CH_3$ | H | 2,4-Difluorphenyl | |
| 665 | 0 | - | - | $CH_3$ | H | 2-Chlorphenyl | |
| 666 | 0 | - | - | $CH_3$ | H | 3-Chlorphenyl | |
| 667 | 0 | - | - | $CH_3$ | H | 4-Chlorphenyl | |
| 668 | 0 | - | - | $CH_3$ | H | 2,4-Dichlorphenyl | |
| 669 | 0 | - | - | $CH_3$ | H | 3,4-Dichlorphenyl | |
| 670 | 0 | - | - | $CH_3$ | H | 2,6-Dichlorphenyl | |
| 671 | 0 | - | - | $CH_3$ | H | 2,4,6-Trichlorphenyl | |
| 672 | 0 | - | - | $CH_3$ | H | 2-Chlor-3-fluorphenyl | |
| 673 | 0 | - | - | $CH_3$ | H | 2-Chlor-4-fluorphenyl | |
| 674 | 0 | - | - | $CH_3$ | H | 2-Chlor-4-fluorphenyl | |
| 675 | 0 | - | - | $CH_3$ | H | 3-Chlor-4-methoxyphenyl | |
| 676 | 0 | - | - | $CH_3$ | H | 2-Chlor-4-methylphenyl | |
| 677 | 0 | - | - | $CH_3$ | H | 3-Chlor-4-hydroxyphenyl | |
| 678 | 0 | - | - | $CH_3$ | H | 2-Chlor-6-fluorphenyl | |
| 679 | 0 | - | - | $CH_3$ | H | 3-Fluor-4-methoxyphenyl | |
| 680 | 0 | - | - | $CH_3$ | H | 2-Bromphenyl | |
| 681 | 0 | - | - | $CH_3$ | H | 3-Bromphenyl | |
| 682 | 0 | - | - | $CH_3$ | H | 4-Bromphenyl | |
| 683 | 0 | - | - | $CH_3$ | H | 2-Brom-4-fluorphenyl | |
| 684 | 0 | - | - | $CH_3$ | H | 2-Brom-4-chlorphenyl | |
| 685 | 0 | - | - | $CH_3$ | H | 2-Methoxyphenyl | |

Fortsetzung Tabelle

| Nr. | n | $R^1$ | $R^2$ | $R^4$ | $R^5$ | Ar | physik. Daten IR ($cm^{-1}$) |
|---|---|---|---|---|---|---|---|
| 686 | 0 | - | - | $CH_3$ | H | 3-Methoxyphenyl | |
| 687 | 0 | - | - | $CH_3$ | H | 4-Methoxyphenyl | |
| 688 | 0 | - | - | $CH_3$ | H | 4-Butoxyphenyl | |
| 689 | 0 | - | - | $CH_3$ | H | 4-n-Propoxyphenyl | |
| 690 | 0 | - | - | $CH_3$ | H | 4-tert-.Butoxyphenyl | |
| 691 | 0 | - | - | $CH_3$ | H | 2-Trifluormethylphenyl | |
| 692 | 0 | - | - | $CH_3$ | H | 3-Trifluormethylphenyl | |
| 693 | 0 | - | - | $CH_3$ | H | 4-Trifluormethylphenyl | |
| 694 | 0 | - | - | $CH_3$ | H | 4-Trifluormethoxyphenyl | |
| 695 | 0 | - | - | $CH_3$ | H | 4-Tetrafluorethoxyphenyl | |
| 696 | 0 | - | - | $CH_3$ | H | 3,4-Dimethoxyphenyl | |
| 697 | 0 | - | - | $CH_3$ | H | 2-Methoxy-4-trifluormethylphenyl | |
| 698 | 0 | - | - | $CH_3$ | H | 4-Phenoxyphenyl | |
| 699 | 0 | - | - | $CH_3$ | H | 4-(4'-Chlorphenoxy)-phenyl | |
| 700 | 0 | - | - | $CH_3$ | H | 2-Nitrophenyl | |
| 701 | 0 | - | - | $CH_3$ | H | 4-Nitrophenyl | |
| 702 | 0 | - | - | $CH_3$ | H | 2-Chlor-4-nitrophenyl | |
| 703 | 0 | - | - | $CH_3$ | H | 4-Hydroxyphenyl | |
| 704 | 0 | - | - | $CH_3$ | H | 2-Hydroxyphenyl | |
| 705 | 0 | - | - | $CH_3$ | $CH_3$ | Phenyl | |
| 706 | 0 | - | - | $CH_3$ | $CH_3$ | 2-Methylphenyl | |
| 707 | 0 | - | - | $CH_3$ | $CH_3$ | 3-Methylphenyl | |
| 708 | 0 | - | - | $CH_3$ | $CH_3$ | 4-Methylphenyl | |
| 709 | 0 | - | - | $CH_3$ | $CH_3$ | 2,4-Dimethylphenyl | |

Fortsetzung Tabelle

| Nr. | n | $R^1$ | $R^2$ | $R^4$ | $R^5$ | Ar | physik. Daten IR ($cm^{-1}$) |
|---|---|---|---|---|---|---|---|
| 710 | 0 | - | - | $CH_3$ | $CH_3$ | 2,6-Dimethylphenyl | |
| 711 | 0 | - | - | $CH_3$ | $CH_3$ | 2,4,6-Trimethylphenyl | |
| 712 | 0 | - | - | $CH_3$ | $CH_3$ | 4-tert.-Butylphenyl | |
| 713 | 0 | - | - | $CH_3$ | $CH_3$ | 1-Naphthyl | |
| 714 | 0 | - | - | $CH_3$ | $CH_3$ | 2-Naphthyl | |
| 715 | 0 | - | - | $CH_3$ | $CH_3$ | 4-Biphenyl | |
| 716 | 0 | - | - | $CH_3$ | $CH_3$ | 2-Fluorphenyl | |
| 717 | 0 | - | - | $CH_3$ | $CH_3$ | 3-Fluorphenyl | |
| 718 | 0 | - | - | $CH_3$ | $CH_3$ | 4-Fluorphenyl | |
| 719 | 0 | - | - | $CH_3$ | $CH_3$ | 2,4-Difluorphenyl | |
| 720 | 0 | - | - | $CH_3$ | $CH_3$ | 2-Chlorphenyl | |
| 721 | 0 | - | - | $CH_3$ | $CH_3$ | 3-Chlorphenyl | |
| 722 | 0 | - | - | $CH_3$ | $CH_3$ | 4-Chlorphenyl | |
| 723 | 0 | - | - | $CH_3$ | $CH_3$ | 2,4-Dichlorphenyl | |
| 724 | 0 | - | - | $CH_3$ | $CH_3$ | 3,4-Dichlorphenyl | |
| 725 | 0 | - | - | $CH_3$ | $CH_3$ | 2,6-Dichlorphenyl | |
| 726 | 0 | - | - | $CH_3$ | $CH_3$ | 2,4,6-Trichlorphenyl | |
| 727 | 0 | - | - | $CH_3$ | $CH_3$ | 2-Chlor-3-fluorphenyl | |
| 728 | 0 | - | - | $CH_3$ | $CH_3$ | 2-Chlor-4-fluorphenyl | |
| 729 | 0 | - | - | $CH_3$ | $CH_3$ | 2-Chlor-4-fluorphenyl | |
| 730 | 0 | - | - | $CH_3$ | $CH_3$ | 3-Chlor-4-methoxyphenyl | |
| 731 | 0 | - | - | $CH_3$ | $CH_3$ | 2-Chlor-4-methylphenyl | |
| 732 | 0 | - | - | $CH_3$ | $CH_3$ | 3-Chlor-4-hydroxyphenyl | |
| 733 | 0 | - | - | $CH_3$ | $CH_3$ | 2-Chlor-6-fluorphenyl | |

Fortsetzung Tabelle

| Nr. | n | $R^1$ | $R^2$ | $R^4$ | $R^5$ | Ar | physik. Daten IR ($cm^{-1}$) |
|---|---|---|---|---|---|---|---|
| 734 | 0 | - | - | $CH_3$ | $CH_3$ | 3-Fluor-4-methoxyphenyl | |
| 735 | 0 | - | - | $CH_3$ | $CH_3$ | 2-Bromphenyl | |
| 736 | 0 | - | - | $CH_3$ | $CH_3$ | 3-Bromphenyl | |
| 737 | 0 | - | - | $CH_3$ | $CH_3$ | 4-Bromphenyl | |
| 738 | 0 | - | - | $CH_3$ | $CH_3$ | 2-Brom-4-fluorphenyl | |
| 739 | 0 | - | - | $CH_3$ | $CH_3$ | 2-Brom-4-chlorphenyl | |
| 740 | 0 | - | - | $CH_3$ | $CH_3$ | 2-Methoxyphenyl | |
| 741 | 0 | - | - | $CH_3$ | $CH_3$ | 3-Methoxyphenyl | |
| 742 | 0 | - | - | $CH_3$ | $CH_3$ | 4-Methoxyphenyl | |
| 743 | 0 | - | - | $CH_3$ | $CH_3$ | 4-Butoxyphenyl | |
| 744 | 0 | - | - | $CH_3$ | $CH_3$ | 4-n-Propoxyphenyl | |
| 745 | 0 | - | - | $CH_3$ | $CH_3$ | 4-tert-.Butoxyphenyl | |
| 746 | 0 | - | - | $CH_3$ | $CH_3$ | 2-Trifluormethylphenyl | |
| 747 | 0 | - | - | $CH_3$ | $CH_3$ | 3-Trifluormethylphenyl | |
| 748 | 0 | - | - | $CH_3$ | $CH_3$ | 4-Trifluormethylphenyl | |
| 749 | 0 | - | - | $CH_3$ | $CH_3$ | 4-Trifluormethoxyphenyl | |
| 750 | 0 | - | - | $CH_3$ | $CH_3$ | 4-Tetrafluorethoxyphenyl | |
| 751 | 0 | - | - | $CH_3$ | $CH_3$ | 3,4-Dimethoxyphenyl | |
| 752 | 0 | - | - | $CH_3$ | $CH_3$ | 2-Methoxy-4-trifluormethylphenyl | |
| 753 | 0 | - | - | $CH_3$ | $CH_3$ | 4-Phenoxyphenyl | |
| 754 | 0 | - | - | $CH_3$ | $CH_3$ | 4-(4'-Chlorphenoxy)-phenyl | |
| 755 | 0 | - | - | $CH_3$ | $CH_3$ | 2-Nitrophenyl | |
| 756 | 0 | - | - | $CH_3$ | $CH_3$ | 4-Nitrophenyl | |
| 757 | 0 | - | - | $CH_3$ | $CH_3$ | 2-Chlor-4-nitrophenyl | |

Fortsetzung Tabelle

| Nr. | n | $R^1$ | $R^2$ | $R^4$ | $R^5$ | Ar | physik. Daten IR ($cm^{-1}$) |
|---|---|---|---|---|---|---|---|
| 758 | 0 | - | - | $CH_3$ | $CH_3$ | 4-Hydroxyphenyl | |
| 759 | 0 | - | - | $CH_3$ | $CH_3$ | 2-Hydroxyphenyl | |
| 760 | 1 | H | H | H | H | 2-Methylphenyl | |
| 761 | 1 | H | H | H | H | 3-Methylphenyl | |
| 762 | 1 | H | H | H | H | 4-Methylphenyl | 3200, 1739, 1700, 1691, 3665, 1406 |
| 763 | 1 | H | H | H | H | 2,4-Dimethylphenyl | |
| 764 | 1 | H | H | H | H | 2,6-Dimethylphenyl | |
| 765 | 1 | H | H | H | H | 2,4,6-Trimethylphenyl | |
| 766 | 1 | H | H | H | H | 4-tert.-Butylphenyl | |
| 767 | 1 | H | H | H | H | 1-Naphthyl | |
| 768 | 1 | H | H | H | H | 2-Naphthyl | |
| 769 | 1 | H | H | H | H | 4-Biphenyl | |
| 770 | 1 | H | H | H | H | 2-Fluorphenyl | |
| 771 | 1 | H | H | H | H | 3-Fluorphenyl | |
| 772 | 1 | H | H | H | H | 4-Fluorphenyl | Fp: 196° 3200, 1738, 1699, 1692, 1518, 1406 |
| 773 | 1 | H | H | H | H | 2,4-Difluorphenyl | |
| 774 | 1 | H | H | H | H | 2-Chlorphenyl | |
| 775 | 1 | H | H | H | H | 3-Chlorphenyl | |
| 776 | 1 | H | H | H | H | 4-Chlorphenyl | |
| 777 | 1 | H | H | H | H | 2,4-Dichlorphenyl | |
| 778 | 1 | H | H | H | H | 3,4-Dichlorphenyl | |
| 779 | 1 | H | H | H | H | 2,6-Dichlorphenyl | |

Fortsetzung Tabelle

| Nr. | n | $R^1$ | $R^2$ | $R^4$ | $R^5$ | Ar | physik. Daten IR ($cm^{-1}$) |
|---|---|---|---|---|---|---|---|
| 780 | 1 | H | H | H | H | 2,4,6-Trichlorphenyl | |
| 781 | 1 | H | H | H | H | 2-Chlor-3-fluorphenyl | |
| 782 | 1 | H | H | H | H | 2-Chlor-4-fluorphenyl | |
| 783 | 1 | H | H | H | H | 2-Chlor-4-fluorphenyl | |
| 784 | 1 | H | H | H | H | 3-Chlor-4-methoxyphenyl | |
| 785 | 1 | H | H | H | H | 2-Chlor-4-methylphenyl | |
| 786 | 1 | H | H | H | H | 3-Chlor-4-hydroxyphenyl | |
| 787 | 1 | H | H | H | H | 2-Chlor-6-fluorphenyl | |
| 788 | 1 | H | H | H | H | 3-Fluor-4-methoxyphenyl | |
| 789 | 1 | H | H | H | H | 2-Bromphenyl | |
| 790 | 1 | H | H | H | H | 3-Bromphenyl | |
| 791 | 1 | H | H | H | H | 4-Bromphenyl | |
| 792 | 1 | H | H | H | H | 2-Brom-4-fluorphenyl | |
| 793 | 1 | H | H | H | H | 2-Brom-4-chlorphenyl | |
| 794 | 1 | H | H | H | H | 2-Methoxyphenyl | |
| 795 | 1 | H | H | H | H | 3-Methoxyphenyl | |
| 796 | 1 | H | H | H | H | 4-Butoxyphenyl | |
| 797 | 1 | H | H | H | H | 4-n-Propoxyphenyl | |
| 798 | 1 | H | H | H | H | 4-tert-.Butoxyphenyl | |
| 799 | 1 | H | H | H | H | 2-Trifluormethylphenyl | |
| 800 | 1 | H | H | H | H | 3-Trifluormethylphenyl | |
| 801 | 1 | H | H | H | H | 4-Trifluormethylphenyl | |
| 802 | 1 | H | H | H | H | 4-Trifluormethoxyphenyl | |
| 803 | 1 | H | H | H | H | 4-Tetrafluorethoxyphenyl | |

Fortsetzung Tabelle

| Nr. | n | $R^1$ | $R^2$ | $R^4$ | $R^5$ | Ar | physik. Daten IR ($cm^{-1}$) |
|---|---|---|---|---|---|---|---|
| 804 | 1 | H | H | H | H | 3,4-Dimethoxyphenyl | |
| 805 | 1 | H | H | H | H | 2-Methoxy-4-trifluormethylphenyl | |
| 806 | 1 | H | H | H | H | 4-Phenoxyphenyl | |
| 807 | 1 | H | H | H | H | 4-(4'-Chlorphenoxy)-phenyl | |
| 808 | 1 | H | H | H | H | 2-Nitrophenyl | |
| 809 | 1 | H | H | H | H | 2-Chlor-4-nitrophenyl | |
| 810 | 1 | H | H | H | H | 4-Hydroxyphenyl | |
| 811 | 1 | H | H | H | H | 2-Hydroxyphenyl | |
| 812 | 1 | H | H | $CH_3$ | H | Phenyl | |
| 813 | 1 | H | H | $CH_3$ | H | 2-Methylphenyl | |
| 814 | 1 | H | H | $CH_3$ | H | 3-Methylphenyl | |
| 815 | 1 | H | H | $CH_3$ | H | 4-Methylphenyl | |
| 816 | 1 | H | H | $CH_3$ | H | 2,4-Dimethylphenyl | |
| 817 | 1 | H | H | $CH_3$ | H | 2,6-Dimethylphenyl | |
| 818 | 1 | H | H | $CH_3$ | H | 2,4,6-Trimethylphenyl | |
| 819 | 1 | H | H | $CH_3$ | H | 4-tert.-Butylphenyl | |
| 820 | 1 | H | H | $CH_3$ | H | 1-Naphthyl | |
| 821 | 1 | H | H | $CH_3$ | H | 2-Naphthyl | |
| 822 | 1 | H | H | $CH_3$ | H | 4-Biphenyl | |
| 823 | 1 | H | H | $CH_3$ | H | 2-Fluorphenyl | |
| 824 | 1 | H | H | $CH_3$ | H | 3-Fluorphenyl | |
| 825 | 1 | H | H | $CH_3$ | H | 4-Fluorphenyl | |
| 826 | 1 | H | H | $CH_3$ | H | 2,4-Difluorphenyl | |
| 827 | 1 | H | H | $CH_3$ | H | 2-Chlorphenyl | |

Fortsetzung Tabelle

| Nr. | n | $R^1$ | $R^2$ | $R^4$ | $R^5$ | Ar | physik. Daten IR ($cm^{-1}$) |
|---|---|---|---|---|---|---|---|
| 828 | 1 | H | H | $CH_3$ | H | 3-Chlorphenyl | |
| 829 | 1 | H | H | $CH_3$ | H | 4-Chlorphenyl | |
| 830 | 1 | H | H | $CH_3$ | H | 2,4-Dichlorphenyl | |
| 831 | 1 | H | H | $CH_3$ | H | 3,4-Dichlorphenyl | |
| 832 | 1 | H | H | $CH_3$ | H | 2,6-Dichlorphenyl | |
| 833 | 1 | H | H | $CH_3$ | H | 2,4,6-Trichlorphenyl | |
| 834 | 1 | H | H | $CH_3$ | H | 2-Chlor-3-fluorphenyl | |
| 835 | 1 | H | H | $CH_3$ | H | 2-Chlor-4-fluorphenyl | |
| 836 | 1 | H | H | $CH_3$ | H | 2-Chlor-4-fluorphenyl | |
| 837 | 1 | H | H | $CH_3$ | H | 3-Chlor-4-methoxyphenyl | |
| 838 | 1 | H | H | $CH_3$ | H | 2-Chlor-4-methylphenyl | |
| 839 | 1 | H | H | $CH_3$ | H | 3-Chlor-4-hydroxyphenyl | |
| 840 | 1 | H | H | $CH_3$ | H | 2-Chlor-6-fluorphenyl | |
| 841 | 1 | H | H | $CH_3$ | H | 3-Fluor-4-methoxyphenyl | |
| 842 | 1 | H | H | $CH_3$ | H | 2-Bromphenyl | |
| 843 | 1 | H | H | $CH_3$ | H | 3-Bromphenyl | |
| 844 | 1 | H | H | $CH_3$ | H | 4-Bromphenyl | |
| 845 | 1 | H | H | $CH_3$ | H | 2-Brom-4-fluorphenyl | |
| 846 | 1 | H | H | $CH_3$ | H | 2-Brom-4-chlorphenyl | |
| 847 | 1 | H | H | $CH_3$ | H | 2-Methoxyphenyl | |
| 848 | 1 | H | H | $CH_3$ | H | 3-Methoxyphenyl | |
| 849 | 1 | H | H | $CH_3$ | H | 4-Methoxyphenyl | |
| 850 | 1 | H | H | $CH_3$ | H | 4-Butoxyphenyl | |
| 851 | 1 | H | H | $CH_3$ | H | 4-n-Propoxyphenyl | |

Fortsetzung Tabelle

| Nr. | n | $R^1$ | $R^2$ | $R^4$ | $R^5$ | Ar | physik. Daten IR ($cm^{-1}$) |
|---|---|---|---|---|---|---|---|
| 852 | 1 | H | H | $CH_3$ | H | 4-tert-.Butoxyphenyl | |
| 853 | 1 | H | H | $CH_3$ | H | 2-Trifluormethylphenyl | |
| 854 | 1 | H | H | $CH_3$ | H | 3-Trifluormethylphenyl | |
| 855 | 1 | H | H | $CH_3$ | H | 4-Trifluormethylphenyl | |
| 856 | 1 | H | H | $CH_3$ | H | 4-Trifluormethoxyphenyl | |
| 857 | 1 | H | H | $CH_3$ | H | 4-Tetrafluorethoxyphenyl | |
| 858 | 1 | H | H | $CH_3$ | H | 3,4-Dimethoxyphenyl | |
| 859 | 1 | H | H | $CH_3$ | H | 2-Methoxy-4-trifluormethylphenyl | |
| 860 | 1 | H | H | $CH_3$ | H | 4-Phenoxyphenyl | |
| 861 | 1 | H | H | $CH_3$ | H | 4-(4'-Chlorphenoxy)-phenyl | |
| 862 | 1 | H | H | $CH_3$ | H | 2-Nitrophenyl | |
| 863 | 1 | H | H | $CH_3$ | H | 4-Nitrophenyl | |
| 864 | 1 | H | H | $CH_3$ | H | 2-Chlor-4-nitrophenyl | |
| 865 | 1 | H | H | $CH_3$ | H | 4-Hydroxyphenyl | |
| 866 | 1 | H | H | $CH_3$ | H | 2-Hydroxyphenyl | |
| 868 | 1 | H | H | $CH_3$ | $CH_3$ | 2-Methylphenyl | |
| 869 | 1 | H | H | $CH_3$ | $CH_3$ | 3-Methylphenyl | |
| 870 | 1 | H | H | $CH_3$ | $CH_3$ | 4-Methylphenyl | |
| 871 | 1 | H | H | $CH_3$ | $CH_3$ | 2,4-Dimethylphenyl | |
| 872 | 1 | H | H | $CH_3$ | $CH_3$ | 2,6-Dimethylphenyl | |
| 873 | 1 | H | H | $CH_3$ | $CH_3$ | 2,4,6-Trimethylphenyl | |
| 874 | 1 | H | H | $CH_3$ | $CH_3$ | 4-tert.-Butylphenyl | |
| 875 | 1 | H | H | $CH_3$ | $CH_3$ | 1-Naphthyl | |

Fortsetzung Tabelle

| Nr. | n | $R^1$ | $R^2$ | $R^4$ | $R^5$ | Ar | physik. Daten IR ($cm^{-1}$) |
|---|---|---|---|---|---|---|---|
| 876 | 1 | H | H | $CH_3$ | $CH_3$ | 2-Naphthyl | |
| 877 | 1 | H | H | $CH_3$ | $CH_3$ | 4-Biphenyl | |
| 878 | 1 | H | H | $CH_3$ | $CH_3$ | 2-Fluorphenyl | |
| 879 | 1 | H | H | $CH_3$ | $CH_3$ | 3-Fluorphenyl | |
| 880 | 1 | H | H | $CH_3$ | $CH_3$ | 4-Fluorphenyl | |
| 881 | 1 | H | H | $CH_3$ | $CH_3$ | 2,4-Difluorphenyl | |
| 882 | 1 | H | H | $CH_3$ | $CH_3$ | 2-Chlorphenyl | |
| 883 | 1 | H | H | $CH_3$ | $CH_3$ | 3-Chlorphenyl | |
| 884 | 1 | H | H | $CH_3$ | $CH_3$ | 4-Chlorphenyl | |
| 885 | 1 | H | H | $CH_3$ | $CH_3$ | 2,4-Dichlorphenyl | |
| 886 | 1 | H | H | $CH_3$ | $CH_3$ | 3,4-Dichlorphenyl | |
| 887 | 1 | H | H | $CH_3$ | $CH_3$ | 2,6-Dichlorphenyl | |
| 888 | 1 | H | H | $CH_3$ | $CH_3$ | 2,4,6-Trichlorphenyl | |
| 889 | 1 | H | H | $CH_3$ | $CH_3$ | 2-Chlor-3-fluorphenyl | |
| 890 | 1 | H | H | $CH_3$ | $CH_3$ | 2-Chlor-4-fluorphenyl | |
| 891 | 1 | H | H | $CH_3$ | $CH_3$ | 2-Chlor-4-fluorphenyl | |
| 892 | 1 | H | H | $CH_3$ | $CH_3$ | 3-Chlor-4-methoxyphenyl | |
| 893 | 1 | H | H | $CH_3$ | $CH_3$ | 2-Chlor-4-methylphenyl | |
| 894 | 1 | H | H | $CH_3$ | $CH_3$ | 3-Chlor-4-hydroxyphenyl | |
| 895 | 1 | H | H | $CH_3$ | $CH_3$ | 2-Chlor-6-fluorphenyl | |
| 896 | 1 | H | H | $CH_3$ | $CH_3$ | 3-Fluor-4-methoxyphenyl | |
| 897 | 1 | H | H | $CH_3$ | $CH_3$ | 2-Bromphenyl | |
| 898 | 1 | H | H | $CH_3$ | $CH_3$ | 3-Bromphenyl | |
| 899 | 1 | H | H | $CH_3$ | $CH_3$ | 4-Bromphenyl | |

Fortsetzung Tabelle

| Nr. | n | $R^1$ | $R^2$ | $R^4$ | $R^5$ | Ar | physik. Daten IR ($cm^{-1}$) |
|---|---|---|---|---|---|---|---|
| 900 | 1 | H | H | $CH_3$ | $CH_3$ | 2-Brom-4-fluorphenyl | |
| 901 | 1 | H | H | $CH_3$ | $CH_3$ | 2-Brom-4-chlorphenyl | |
| 902 | 1 | H | H | $CH_3$ | $CH_3$ | 2-Methoxyphenyl | |
| 903 | 1 | H | H | $CH_3$ | $CH_3$ | 3-Methoxyphenyl | |
| 904 | 1 | H | H | $CH_3$ | $CH_3$ | 4-Methoxyphenyl | |
| 905 | 1 | H | H | $CH_3$ | $CH_3$ | 4-Butoxyphenyl | |
| 906 | 1 | H | H | $CH_3$ | $CH_3$ | 4-n-Propoxyphenyl | |
| 907 | 1 | H | H | $CH_3$ | $CH_3$ | 4-tert-.Butoxyphenyl | |
| 908 | 1 | H | H | $CH_3$ | $CH_3$ | 2-Trifluormethylphenyl | |
| 909 | 1 | H | H | $CH_3$ | $CH_3$ | 3-Trifluormethylphenyl | |
| 910 | 1 | H | H | $CH_3$ | $CH_3$ | 4-Trifluormethylphenyl | |
| 911 | 1 | H | H | $CH_3$ | $CH_3$ | 4-Trifluormethoxyphenyl | |
| 912 | 1 | H | H | $CH_3$ | $CH_3$ | 4-Tetrafluorethoxyphenyl | |
| 913 | 1 | H | H | $CH_3$ | $CH_3$ | 3,4-Dimethoxyphenyl | |
| 914 | 1 | H | H | $CH_3$ | $CH_3$ | 2-Methoxy-4-trifluormethylphenyl | |
| 915 | 1 | H | H | $CH_3$ | $CH_3$ | 4-Phenoxyphenyl | |
| 916 | 1 | H | H | $CH_3$ | $CH_3$ | 4-(4'-Chlorphenoxy)-phenyl | |
| 917 | 1 | H | H | $CH_3$ | $CH_3$ | 2-Nitrophenyl | |
| 918 | 1 | H | H | $CH_3$ | $CH_3$ | 4-Nitrophenyl | |
| 919 | 1 | H | H | $CH_3$ | $CH_3$ | 2-Chlor-4-nitrophenyl | |
| 920 | 1 | H | H | $CH_3$ | $CH_3$ | 4-Hydroxyphenyl | |
| 921 | 1 | H | H | $CH_3$ | $CH_3$ | 2-Hydroxyphenyl | |
| 922 | 1 | $CH_3$ | $CH_3$ | H | H | Phenyl | |
| 923 | 1 | $CH_3$ | $CH_3$ | H | H | 2-Methylphenyl | |

Fortsetzung Tabelle

| Nr. | n | $R^1$ | $R^2$ | $R^4$ | $R^5$ | Ar | physik. Daten IR ($cm^{-1}$) |
|---|---|---|---|---|---|---|---|
| 924 | 1 | $CH_3$ | $CH_3$ | H | H | 3-Methylphenyl | |
| 925 | 1 | $CH_3$ | $CH_3$ | H | H | 4-Methylphenyl | |
| 926 | 1 | $CH_3$ | $CH_3$ | H | H | 2,4-Dimethylphenyl | |
| 927 | 1 | $CH_3$ | $CH_3$ | H | H | 2,6-Dimethylphenyl | |
| 928 | 1 | $CH_3$ | $CH_3$ | H | H | 2,4,6-Trimethylphenyl | |
| 929 | 1 | $CH_3$ | $CH_3$ | H | H | 4-tert.-Butylphenyl | |
| 930 | 1 | $CH_3$ | $CH_3$ | H | H | 1-Naphthyl | |
| 931 | 1 | $CH_3$ | $CH_3$ | H | H | 2-Naphthyl | |
| 932 | 1 | $CH_3$ | $CH_3$ | H | H | 4-Biphenyl | |
| 933 | 1 | $CH_3$ | $CH_3$ | H | H | 2-Fluorphenyl | |
| 934 | 1 | $CH_3$ | $CH_3$ | H | H | 3-Fluorphenyl | |
| 935 | 1 | $CH_3$ | $CH_3$ | H | H | 4-Fluorphenyl | |
| 936 | 1 | $CH_3$ | $CH_3$ | H | H | 2,4-Difluorphenyl | |
| 937 | 1 | $CH_3$ | $CH_3$ | H | H | 2-Chlorphenyl | |
| 938 | 1 | $CH_3$ | $CH_3$ | H | H | 3-Chlorphenyl | |
| 939 | 1 | $CH_3$ | $CH_3$ | H | H | 4-Chlorphenyl | |
| 940 | 1 | $CH_3$ | $CH_3$ | H | H | 2,4-Dichlorphenyl | |
| 941 | 1 | $CH_3$ | $CH_3$ | H | H | 3,4-Dichlorphenyl | |
| 942 | 1 | $CH_3$ | $CH_3$ | H | H | 2,6-Dichlorphenyl | |
| 943 | 1 | $CH_3$ | $CH_3$ | H | H | 2,4,6-Trichlorphenyl | |
| 944 | 1 | $CH_3$ | $CH_3$ | H | H | 2-Chlor-3-fluorphenyl | |
| 945 | 1 | $CH_3$ | $CH_3$ | H | H | 2-Chlor-4-fluorphenyl | |
| 946 | 1 | $CH_3$ | $CH_3$ | H | H | 2-Chlor-4-fluorphenyl | |
| 947 | 1 | $CH_3$ | $CH_3$ | H | H | 3-Chlor-4-methoxyphenyl | |

Fortsetzung Tabelle

| Nr. | n | $R^1$ | $R^2$ | $R^4$ | $R^5$ | Ar | physik. Daten IR ($cm^{-1}$) |
|---|---|---|---|---|---|---|---|
| 948 | 1 | $CH_3$ | $CH_3$ | H | H | 2-Chlor-4-methylphenyl | |
| 949 | 1 | $CH_3$ | $CH_3$ | H | H | 3-Chlor-4-hydroxyphenyl | |
| 950 | 1 | $CH_3$ | $CH_3$ | H | H | 2-Chlor-6-fluorphenyl | |
| 951 | 1 | $CH_3$ | $CH_3$ | H | H | 3-Fluor-4-methoxyphenyl | |
| 952 | 1 | $CH_3$ | $CH_3$ | H | H | 2-Bromphenyl | |
| 953 | 1 | $CH_3$ | $CH_3$ | H | H | 3-Bromphenyl | |
| 954 | 1 | $CH_3$ | $CH_3$ | H | H | 4-Bromphenyl | |
| 955 | 1 | $CH_3$ | $CH_3$ | H | H | 2-Brom-4-fluorphenyl | |
| 956 | 1 | $CH_3$ | $CH_3$ | H | H | 2-Brom-4-chlorphenyl | |
| 957 | 1 | $CH_3$ | $CH_3$ | H | H | 2-Methoxyphenyl | |
| 958 | 1 | $CH_3$ | $CH_3$ | H | H | 3-Methoxyphenyl | |
| 959 | 1 | $CH_3$ | $CH_3$ | H | H | 4-Methoxyphenyl | |
| 960 | 1 | $CH_3$ | $CH_3$ | H | H | 4-Butoxyphenyl | |
| 961 | 1 | $CH_3$ | $CH_3$ | H | H | 4-n-Propoxyphenyl | |
| 962 | 1 | $CH_3$ | $CH_3$ | H | H | 4-tert-.Butoxyphenyl | |
| 963 | 1 | $CH_3$ | $CH_3$ | H | H | 2-Trifluormethylphenyl | |
| 964 | 1 | $CH_3$ | $CH_3$ | H | H | 3-Trifluormethylphenyl | |
| 965 | 1 | $CH_3$ | $CH_3$ | H | H | 4-Trifluormethylphenyl | |
| 966 | 1 | $CH_3$ | $CH_3$ | H | H | 4-Trifluormethoxyphenyl | |
| 967 | 1 | $CH_3$ | $CH_3$ | H | H | 4-Tetrafluorethoxyphenyl | |
| 968 | 1 | $CH_3$ | $CH_3$ | H | H | 3,4-Dimethoxyphenyl | |
| 969 | 1 | $CH_3$ | $CH_3$ | H | H | 2-Methoxy-4-trifluormethylphenyl | |
| 970 | 1 | $CH_3$ | $CH_3$ | H | H | 4-Phenoxyphenyl | |
| 971 | 1 | $CH_3$ | $CH_3$ | H | H | 4-(4'-Chlorphenoxy)-phenyl | |

Fortsetzung Tabelle

| Nr. | n | $R^1$ | $R^2$ | $R^4$ | $R^5$ | Ar | physik. Daten IR ($cm^{-1}$) |
|---|---|---|---|---|---|---|---|
| 972 | 1 | $CH_3$ | $CH_3$ | H | H | 2-Nitrophenyl | |
| 973 | 1 | $CH_3$ | $CH_3$ | H | H | 4-Nitrophenyl | |
| 974 | 1 | $CH_3$ | $CH_3$ | H | H | 2-Chlor-4-nitrophenyl | |
| 975 | 1 | $CH_3$ | $CH_3$ | H | H | 4-Hydroxyphenyl | |
| 976 | 1 | $CH_3$ | $CH_3$ | H | H | 2-Hydroxyphenyl | |
| 977 | 1 | $CH_3$ | $CH_3$ | $CH_3$ | H | Phenyl | |
| 978 | 1 | $CH_3$ | $CH_3$ | $CH_3$ | H | 2-Methylphenyl | |
| 979 | 1 | $CH_3$ | $CH_3$ | $CH_3$ | H | 3-Methylphenyl | |
| 980 | 1 | $CH_3$ | $CH_3$ | $CH_3$ | H | 4-Methylphenyl | |
| 981 | 1 | $CH_3$ | $CH_3$ | $CH_3$ | H | 2,4-Dimethylphenyl | |
| 982 | 1 | $CH_3$ | $CH_3$ | $CH_3$ | H | 2,6-Dimethylphenyl | |
| 983 | 1 | $CH_3$ | $CH_3$ | $CH_3$ | H | 2,4,6-Trimethylphenyl | |
| 984 | 1 | $CH_3$ | $CH_3$ | $CH_3$ | H | 4-tert.-Butylphenyl | |
| 985 | 1 | $CH_3$ | $CH_3$ | $CH_3$ | H | 1-Naphthyl | |
| 986 | 1 | $CH_3$ | $CH_3$ | $CH_3$ | H | 2-Naphthyl | |
| 987 | 1 | $CH_3$ | $CH_3$ | $CH_3$ | H | 4-Biphenyl | |
| 988 | 1 | $CH_3$ | $CH_3$ | $CH_3$ | H | 2-Fluorphenyl | |
| 989 | 1 | $CH_3$ | $CH_3$ | $CH_3$ | H | 3-Fluorphenyl | |
| 990 | 1 | $CH_3$ | $CH_3$ | $CH_3$ | H | 4-Fluorphenyl | |
| 991 | 1 | $CH_3$ | $CH_3$ | $CH_3$ | H | 2,4-Difluorphenyl | |
| 992 | 1 | $CH_3$ | $CH_3$ | $CH_3$ | H | 2-Chlorphenyl | |
| 993 | 1 | $CH_3$ | $CH_3$ | $CH_3$ | H | 3-Chlorphenyl | |
| 994 | 1 | $CH_3$ | $CH_3$ | $CH_3$ | H | 4-Chlorphenyl | |
| 995 | 1 | $CH_3$ | $CH_3$ | $CH_3$ | H | 2,4-Dichlorphenyl | |

Fortsetzung Tabelle

| Nr. | n | $R^1$ | $R^2$ | $R^4$ | $R^5$ | Ar | physik. Daten IR ($cm^{-1}$) |
|---|---|---|---|---|---|---|---|
| 996 | 1 | $CH_3$ | $CH_3$ | $CH_3$ | H | 3,4-Dichlorphenyl | |
| 997 | 1 | $CH_3$ | $CH_3$ | $CH_3$ | H | 2,6-Dichlorphenyl | |
| 998 | 1 | $CH_3$ | $CH_3$ | $CH_3$ | H | 2,4,6-Trichlorphenyl | |
| 999 | 1 | $CH_3$ | $CH_3$ | $CH_3$ | H | 2-Chlor-3-fluorphenyl | |
| 1000 | 1 | $CH_3$ | $CH_3$ | $CH_3$ | H | 2-Chlor-4-fluorphenyl | |
| 1001 | 1 | $CH_3$ | $CH_3$ | $CH_3$ | H | 2-Chlor-4-fluorphenyl | |
| 1002 | 1 | $CH_3$ | $CH_3$ | $CH_3$ | H | 3-Chlor-4-methoxyphenyl | |
| 1003 | 1 | $CH_3$ | $CH_3$ | $CH_3$ | H | 2-Chlor-4-methylphenyl | |
| 1004 | 1 | $CH_3$ | $CH_3$ | $CH_3$ | H | 3-Chlor-4-hydroxyphenyl | |
| 1005 | 1 | $CH_3$ | $CH_3$ | $CH_3$ | H | 2-Chlor-6-fluorphenyl | |
| 1006 | 1 | $CH_3$ | $CH_3$ | $CH_3$ | H | 3-Fluor-4-methoxyphenyl | |
| 1007 | 1 | $CH_3$ | $CH_3$ | $CH_3$ | H | 2-Bromphenyl | |
| 1008 | 1 | $CH_3$ | $CH_3$ | $CH_3$ | H | 3-Bromphenyl | |
| 1009 | 1 | $CH_3$ | $CH_3$ | $CH_3$ | H | 4-Bromphenyl | |
| 1010 | 1 | $CH_3$ | $CH_3$ | $CH_3$ | H | 2-Brom-4-fluorphenyl | |
| 1011 | 1 | $CH_3$ | $CH_3$ | $CH_3$ | H | 2-Brom-4-chlorphenyl | |
| 1012 | 1 | $CH_3$ | $CH_3$ | $CH_3$ | H | 2-Methoxyphenyl | |
| 1013 | 1 | $CH_3$ | $CH_3$ | $CH_3$ | H | 3-Methoxyphenyl | |
| 1014 | 1 | $CH_3$ | $CH_3$ | $CH_3$ | H | 4-Methoxyphenyl | |
| 1015 | 1 | $CH_3$ | $CH_3$ | $CH_3$ | H | 4-Butoxyphenyl | |
| 1016 | 1 | $CH_3$ | $CH_3$ | $CH_3$ | H | 4-n-Propoxyphenyl | |
| 1017 | 1 | $CH_3$ | $CH_3$ | $CH_3$ | H | 4-tert-.Butoxyphenyl | |
| 1018 | 1 | $CH_3$ | $CH_3$ | $CH_3$ | H | 2-Trifluormethylphenyl | |
| 1019 | 1 | $CH_3$ | $CH_3$ | $CH_3$ | H | 3-Trifluormethylphenyl | |

Fortsetzung Tabelle

| Nr. | n | $R^1$ | $R^2$ | $R^4$ | $R^5$ | Ar | physik. Daten IR ($cm^{-1}$) |
|---|---|---|---|---|---|---|---|
| 1020 | 1 | $CH_3$ | $CH_3$ | $CH_3$ | H | 4-Trifluormethylphenyl | |
| 1021 | 1 | $CH_3$ | $CH_3$ | $CH_3$ | H | 4-Trifluormethoxyphenyl | |
| 1022 | 1 | $CH_3$ | $CH_3$ | $CH_3$ | H | 4-Tetrafluorethoxyphenyl | |
| 1023 | 1 | $CH_3$ | $CH_3$ | $CH_3$ | H | 3,4-Dimethoxyphenyl | |
| 1024 | 1 | $CH_3$ | $CH_3$ | $CH_3$ | H | 2-Methoxy-4-trifluormethylphenyl | |
| 1025 | 1 | $CH_3$ | $CH_3$ | $CH_3$ | H | 4-Phenoxyphenyl | |
| 1026 | 1 | $CH_3$ | $CH_3$ | $CH_3$ | H | 4-(4'-Chlorphenoxy)-phenyl | |
| 1027 | 1 | $CH_3$ | $CH_3$ | $CH_3$ | H | 2-Nitrophenyl | |
| 1028 | 1 | $CH_3$ | $CH_3$ | $CH_3$ | H | 4-Nitrophenyl | |
| 1029 | 1 | $CH_3$ | $CH_3$ | $CH_3$ | H | 2-Chlor-4-nitrophenyl | |
| 1030 | 1 | $CH_3$ | $CH_3$ | $CH_3$ | H | 4-Hydroxyphenyl | |
| 1031 | 1 | $CH_3$ | $CH_3$ | $CH_3$ | H | 2-Hydroxyphenyl | |
| 1032 | 1 | $CH_3$ | $CH_3$ | $CH_3$ | $CH_3$ | Phenyl | |
| 1033 | 1 | $CH_3$ | $CH_3$ | $CH_3$ | $CH_3$ | 2-Methylphenyl | |
| 1034 | 1 | $CH_3$ | $CH_3$ | $CH_3$ | $CH_3$ | 3-Methylphenyl | |
| 1035 | 1 | $CH_3$ | $CH_3$ | $CH_3$ | $CH_3$ | 4-Methylphenyl | |
| 1036 | 1 | $CH_3$ | $CH_3$ | $CH_3$ | $CH_3$ | 2,4-Dimethylphenyl | |
| 1037 | 1 | $CH_3$ | $CH_3$ | $CH_3$ | $CH_3$ | 2,6-Dimethylphenyl | |
| 1038 | 1 | $CH_3$ | $CH_3$ | $CH_3$ | $CH_3$ | 2,4,6-Trimethylphenyl | |
| 1039 | 1 | $CH_3$ | $CH_3$ | $CH_3$ | $CH_3$ | 4-tert.-Butylphenyl | |
| 1040 | 1 | $CH_3$ | $CH_3$ | $CH_3$ | $CH_3$ | 1-Naphthyl | |
| 1041 | 1 | $CH_3$ | $CH_3$ | $CH_3$ | $CH_3$ | 2-Naphthyl | |
| 1042 | 1 | $CH_3$ | $CH_3$ | $CH_3$ | $CH_3$ | 4-Biphenyl | |
| 1043 | 1 | $CH_3$ | $CH_3$ | $CH_3$ | $CH_3$ | 2-Fluorphenyl | |

Fortsetzung Tabelle

| Nr. | n | $R^1$ | $R^2$ | $R^4$ | $R^5$ | Ar | physik. Daten IR ($cm^{-1}$) |
|---|---|---|---|---|---|---|---|
| 1044 | 1 | $CH_3$ | $CH_3$ | $CH_3$ | $CH_3$ | 3-Fluorphenyl | |
| 1045 | 1 | $CH_3$ | $CH_3$ | $CH_3$ | $CH_3$ | 4-Fluorphenyl | |
| 1046 | 1 | $CH_3$ | $CH_3$ | $CH_3$ | $CH_3$ | 2,4-Difluorphenyl | |
| 1047 | 1 | $CH_3$ | $CH_3$ | $CH_3$ | $CH_3$ | 2-Chlorphenyl | |
| 1048 | 1 | $CH_3$ | $CH_3$ | $CH_3$ | $CH_3$ | 3-Chlorphenyl | |
| 1049 | 1 | $CH_3$ | $CH_3$ | $CH_3$ | $CH_3$ | 4-Chlorphenyl | |
| 1050 | 1 | $CH_3$ | $CH_3$ | $CH_3$ | $CH_3$ | 2,4-Dichlorphenyl | |
| 1051 | 1 | $CH_3$ | $CH_3$ | $CH_3$ | $CH_3$ | 3,4-Dichlorphenyl | |
| 1052 | 1 | $CH_3$ | $CH_3$ | $CH_3$ | $CH_3$ | 2,6-Dichlorphenyl | |
| 1053 | 1 | $CH_3$ | $CH_3$ | $CH_3$ | $CH_3$ | 2,4,6-Trichlorphenyl | |
| 1054 | 1 | $CH_3$ | $CH_3$ | $CH_3$ | $CH_3$ | 2-Chlor-3-fluorphenyl | |
| 1055 | 1 | $CH_3$ | $CH_3$ | $CH_3$ | $CH_3$ | 2-Chlor-4-fluorphenyl | |
| 1056 | 1 | $CH_3$ | $CH_3$ | $CH_3$ | $CH_3$ | 2-Chlor-4-fluorphenyl | |
| 1057 | 1 | $CH_3$ | $CH_3$ | $CH_3$ | $CH_3$ | 3-Chlor-4-methoxyphenyl | |
| 1058 | 1 | $CH_3$ | $CH_3$ | $CH_3$ | $CH_3$ | 2-Chlor-4-methylphenyl | |
| 1059 | 1 | $CH_3$ | $CH_3$ | $CH_3$ | $CH_3$ | 3-Chlor-4-hydroxyphenyl | |
| 1060 | 1 | $CH_3$ | $CH_3$ | $CH_3$ | $CH_3$ | 2-Chlor-6-fluorphenyl | |
| 1061 | 1 | $CH_3$ | $CH_3$ | $CH_3$ | $CH_3$ | 3-Fluor-4-methoxyphenyl | |
| 1062 | 1 | $CH_3$ | $CH_3$ | $CH_3$ | $CH_3$ | 2-Bromphenyl | |
| 1063 | 1 | $CH_3$ | $CH_3$ | $CH_3$ | $CH_3$ | 3-Bromphenyl | |
| 1064 | 1 | $CH_3$ | $CH_3$ | $CH_3$ | $CH_3$ | 4-Bromphenyl | |
| 1065 | 1 | $CH_3$ | $CH_3$ | $CH_3$ | $CH_3$ | 2-Brom-4-fluorphenyl | |
| 1066 | 1 | $CH_3$ | $CH_3$ | $CH_3$ | $CH_3$ | 2-Brom-4-chlorphenyl | |
| 1067 | 1 | $CH_3$ | $CH_3$ | $CH_3$ | $CH_3$ | 2-Methoxyphenyl | |

Fortsetzung Tabelle

| Nr. | n | $R^1$ | $R^2$ | $R^4$ | $R^5$ | Ar | physik. Daten IR ($cm^{-1}$) |
|---|---|---|---|---|---|---|---|
| 1068 | 1 | $CH_3$ | $CH_3$ | $CH_3$ | $CH_3$ | 3-Methoxyphenyl | |
| 1069 | 1 | $CH_3$ | $CH_3$ | $CH_3$ | $CH_3$ | 4-Methoxyphenyl | |
| 1070 | 1 | $CH_3$ | $CH_3$ | $CH_3$ | $CH_3$ | 4-Butoxyphenyl | |
| 1071 | 1 | $CH_3$ | $CH_3$ | $CH_3$ | $CH_3$ | 4-n-Propoxyphenyl | |
| 1072 | 1 | $CH_3$ | $CH_3$ | $CH_3$ | $CH_3$ | 4-tert-.Butoxyphenyl | |
| 1073 | 1 | $CH_3$ | $CH_3$ | $CH_3$ | $CH_3$ | 2-Trifluormethylphenyl | |
| 1074 | 1 | $CH_3$ | $CH_3$ | $CH_3$ | $CH_3$ | 3-Trifluormethylphenyl | |
| 1075 | 1 | $CH_3$ | $CH_3$ | $CH_3$ | $CH_3$ | 4-Trifluormethylphenyl | |
| 1076 | 1 | $CH_3$ | $CH_3$ | $CH_3$ | $CH_3$ | 4-Trifluormethoxyphenyl | |
| 1077 | 1 | $CH_3$ | $CH_3$ | $CH_3$ | $CH_3$ | 4-Tetrafluorethoxyphenyl | |
| 1088 | 1 | $CH_3$ | $CH_3$ | $CH_3$ | $CH_3$ | 3,4-Dimethoxyphenyl | |
| 1089 | 1 | $CH_3$ | $CH_3$ | $CH_3$ | $CH_3$ | 2-Methoxy-4-trifluormethylphenyl | |
| 1090 | 1 | $CH_3$ | $CH_3$ | $CH_3$ | $CH_3$ | 4-Phenoxyphenyl | |
| 1091 | 1 | $CH_3$ | $CH_3$ | $CH_3$ | $CH_3$ | 4-(4'-Chlorphenoxy)-phenyl | |
| 1092 | 1 | $CH_3$ | $CH_3$ | $CH_3$ | $CH_3$ | 2-Nitrophenyl | |
| 1093 | 1 | $CH_3$ | $CH_3$ | $CH_3$ | $CH_3$ | 4-Nitrophenyl | |
| 1094 | 1 | $CH_3$ | $CH_3$ | $CH_3$ | $CH_3$ | 2-Chlor-4-nitrophenyl | |
| 1095 | 1 | $CH_3$ | $CH_3$ | $CH_3$ | $CH_3$ | 4-Hydroxyphenyl | |
| 1096 | 1 | $CH_3$ | $CH_3$ | $CH_3$ | $CH_3$ | 2-Hydroxyphenyl | |

Die N-Fumaramidsäurederivate eignen sich als Fungizide.

Die erfindungsgemäßen fungiziden Verbindungen bzw. die sie enthaltenden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwecken; sie sollten in jedem Fall möglichst die feinste Verteilung der erfindungsgemäßen Wirkstoffe gewährleisten.

Normalerweise werden die Pflanzen mit den Wirkstoffen besprüht oder bestäubt oder die Samen der Pflanzen mit den Wirkstoffen behandelt.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin), Dimethylformamid und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin-Sulfitablaugen und Methylcellulose.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Beispiele für solche Zubereitungen sind:

I. eine Lösung aus 90 Gew.-Teilen der Verbindung Nr. 183 und 10 Gew.-Teilen N-Methyl-α-pyrrolidon, die zur Anwendung in Form kleinster Tropfen geeignet ist;

II. eine Mischung aus 20 Gew.-Teilen der Verbindung Nr. 166, 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure, 5 Gew.-Teilen des Anlagerungsproduktes von 40 Mol Ethylenoxid an 1 Mol Ricinusöl; durch feines Verteilen der Lösung in Wasser erhält man eine Dispersion.

III. eine wäßrige Dispersion aus 20 Gew.-Teilen der Verbindung Nr. 203, 40 Gew.-Teilen Cyclohexanon, 30 Gew.-Teilen Isobutanol, 20 Gew.-Teilen des Anlagerungsproduktes von 40 mol Ethylenoxid an 1 mol Ricinusöl;

IV. eine wäßrige Dispersion aus 20 Gew.-Teilen der Verbindung Nr. 204, 25 Gew.-Teilen Cyclohexanol, 65 Gew.-Teilen einer Mineralölfraktion vom Siedepunkt 210 bis 280 °C und 10 Gew.-Teilen des Anlagerungsproduktes von 40 mol Ethylenoxid an 1 mol Ricinusöl;

V. eine in einer Hammermühle vermahlene Mischung aus 80 Gew.-Teilen der Verbindung Nr. 219, 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel; durch feines Verteilen der Mischung in Wasser erhält man eine Spritzbrühe;

VI. eine innige Mischung aus 3 Gew.-Teilen der Verbindung Nr. 224 und 97 Gew.-Teilen feinteiligem Kaolin; dieses Stäubemittel enthält 3 Gew.-% Wirkstoff;

VII. eine innige Mischung aus 30 Gew.-Teilen der Verbindung Nr. 234, 92 Gew.-Teilen pulverförmigem Kieselsäuregel und 8 Gew.-Teilen Paraffinöl, das auf die Oberfläche dieses Kieselsäuregels gesprüht wurde; diese Aufbereitung gibt dem Wirkstoff eine gute Haftfähigkeit;

VIII. eine stabile wäßrige Dispersion aus 40 Gew.-Teilen der Verbindung Nr. 327, 10 Gew.-Teilen des Natriumsalzes eines Phenolsulfonsäure-harnstoff-formaldehyd-Kondensates, 2 Gew.-Teilen Kieselgel und 48 Gew.-Teilen Wasser, die weiter verdünnt werden kann;

IX. eine stabile ölige Dispersion aus 20 Gew.-Teilen der Verbindung Nr. 753, 2 Gew.-Teilen des Calciumsalzes der Dodecylbenzolsulfonsäure, 8 Gew.-Teilen Fettalkohol-polyglykolether, 20

Gew.-Teilen des Natriumsalzes eines Phenolsulfonsäure-harnstoff-formaldehyd-Kondensates und 68 Gew.-Teilen eines paraffinischen Mineralöls.

Die N-Fumaramidsäurederivate zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Die Verbindungen werden angewendet, indem man die Pilze oder die vor Pilzbefall zu schützenden Saatgüter, Pflanzen, Materialien oder den Erdboden mit einer fungizid wirksamen Menge der Wirkstoffe behandelt.

Die Anwendung erfolgt vor oder nach der Infektion der Materialien, Pflanzen oder Samen durch die Pilze.

Speziell eignen sich die Verbindungen zur Bekämpfung folgender Pflanzenkrankheiten:

Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die neuen Verbindungen können auch im Materialschutz (Holzschutz) eingesetzt werden, z.B. gegen Paecilomyces variotii.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff pro ha.

Bei der Saatgutbehandlung werden im allgemeinen Wirkstoffmengen von 0,001 bis 50 g, vorzugsweise 0,01 bis 10 g je Kilogramm Saatgut benötigt.

Die erfindungsgemäßen Mittel können in der Anwendungsform als Fungizide auch zusammen mit anderen Wirkstoffen vorliegen, z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums.

Als Vergleichswirkstoff wurde N-(Phenylacetyl)-fumarsäureamid (A) benutzt.

Anwendungsbeispiel 1

Wirksamkeit gegen Plasmopara viticola

Blätter von Topfreben der Sorte "Müller Thurgau" wurden mit wäßriger Spritzbrühe, die 80 % Wirkstoff und 20 % Emulgiermittel in der Trockensubstanz enthielt, besprüht. Um die Wirkungsdauer der Wirkstoffe beurteilen zu können, wurden die Pflanzen nach dem Antrocknen des Spritzbelages 8 Tage im Gewächshaus aufgestellt. Erst dann wurden die Blätter mit einer Zoosporenaufschwemmung von Plasmopara viticola (Rebenperonospora) infiziert. Danach wurden die Rhen zunächst für 48 Stunden in einer wasserdampfgesättigten Kammer bei 24°C und anschließend für 5 Tage in einem Gewächshaus bei Temperaturen zwischen 20 und 30°C aufgestellt. Nach dieser Zeit wurden die Pflanzen zur Beschleunigung des Sporangienträgerausbruchs abermals für 16 Stunden in der feuchten Kammer aufgestellt. Dann erfolgte die Beurteilung des Ausmaßes des Pilzausbruchs auf den Blattunterseiten.

Das Ergebnis zeigt, daß die Wirkstoffe 166, 183, 193, 203, 219 und 224 bei der Anwendung als 0,025-%ige (Gew.-%) Spritzbrühe eine bessere fungizide Wirkung zeigen (80 %) als der bekannte Vergleichswirkstoff A (0 %).

## Patentansprüche

**1.** N-Fumaramidsäure-Derivate der Formel 1

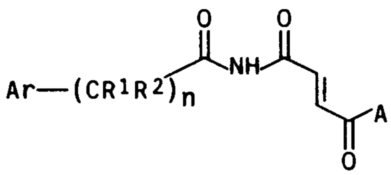

in welcher

- A für O-$R^3$ oder $NR^4R^5$ steht, wobei
- $R^3$, $R^4$, $R^5$ unabhängig voneinander Wasserstoff, $C_1$-$C_6$-Alkyl, $C_1$-$C_4$-Halogenalkyl, $C_2$-$C_6$-Alkenyl, $C_2$-$C_6$-Alkinyl, Aralkyl mit 1-4 Kohlenstoffatomen im Alkylteil und der Arylrest ggf. ein- bis dreifach substituiert ist durch Substituenten aus der Gruppe $C_1$-$C_4$-Alkyl, $C_1$-$C_4$-Alkoxy, $C_1$-$C_4$-Halogenalkoxy, $C_1$-$C_4$-Halogenalkyl, Halogen, Cyan oder Nitro, bedeuten
- $R^1$, $R^2$ unabhängig voneinander Wasserstoff, $C_1$-$C_6$-Alkyl oder $R^1$ und $R^2$, zusammen eine Methylenkette mit 2 bis 6 Methylengruppen bedeuten,
- Ar einen ein- oder zweikernigen Arylrest bedeutet, der ggf. ein bis dreifach substituiert ist durch $C_1$-$C_4$-Alkyl, $C_1$-$C_4$-Alkoxy, $C_1$-$C_4$-Halogenalkyl, Phenoxy, Halogenphenoxy, Benzyloxy, Phenyl, $C_1$-$C_4$-Halogenalkoxy,Hydroxy, Halogen oder Nitro und
- n für 0 - 3 steht, außer den Verbindungen, in denen A $NH_2$ und Ar Phenyl, 4-Nitrophenyl bedeutet und außer den Verbindungen, in denen A Ethoxy oder $N(CH_3)_2$ und Ar Phenyl bedeutet.

**2.** Fungizid, enthaltend einen inerten Trägerstoff und eine fungizid wirksame Menge eines N-Fumaramidsäurederivats der Formel

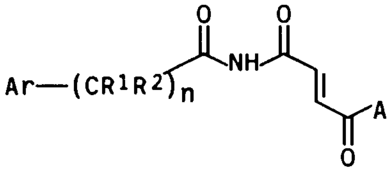

in welcher

- A für O-$R^3$ oder $NR^4R^5$ steht, wobei
- $R^3$, $R^4$, $R^5$ unabhängig voneinander Wasserstoff, $C_1$-$C_6$-Alkyl, $C_1$-$C_4$-Halogenalkyl, $C_2$-$C_6$-Alkenyl, $C_2$-$C_6$-Alkinyl, Aralkyl mit 1-4 Kohlenstoffatomen im Alkylteil und der Arylrest ggf. ein- bis dreifach substituiert ist durch Substituenten aus der Gruppe $C_1$-$C_4$-Alkyl, $C_1$-$C_4$-Alkoxy, $C_1$-$C_4$-Halogenalkoxy, $C_1$-$C_4$-Halogenalkyl, Halogen, Cyan oder Nitro, bedeuten
- $R^1$, $R^2$ unabhängig voneinander Wasserstoff, $C_1$-$C_6$-Alkyl oder $R^1$ und $R^2$, zusammen eine Methylenkette mit 2 bis 6 Methylengruppen bedeuten,
- Ar einen ein- oder zweikernigen Arylrest bedeutet, der ggf. ein bis dreifach substituiert ist durch $C_1$-$C_4$-Alkyl, $C_1$-$C_4$-Alkoxy, $C_1$-$C_4$-Halogenalkyl, Phenoxy, Halogenphenoxy, Benzyloxy, Phenyl, $C_1$-$C_4$-Halogenalkoxy,Hydroxy, Halogen oder Nitro und

n für 0 - 3 steht.

3. Verfahren zur Bekämpfung von Pilzen, dadurch gekennzeichnet, daß man die Pilze oder die vom Pilzbefall bedrohten Materialien, Pflanzen, Böden oder Saatgüter mit einer fungizid wirksamen Menge eines N-Fumaramidsäurederivats der Formel

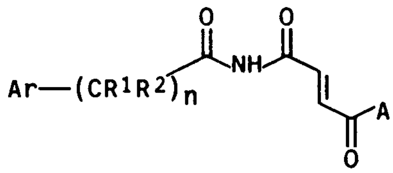

in welcher

A für O-$R^3$ oder $NR^4R^5$ steht, wobei

$R^3$, $R^4$, $R^5$ unabhängig voneinander Wasserstoff, $C_1$-$C_6$-Alkyl, $C_1$-$C_4$-Halogenalkyl, $C_2$-$C_6$-Alkenyl, $C_2$-$C_6$-Alkinyl, Aralkyl mit 1-4 Kohlenstoffatomen im Alkylteil und der Arylrest ggf. ein- bis dreifach substituiert ist durch Substituenten aus der Gruppe $C_1$-$C_4$-Alkyl, $C_1$-$C_4$-Alkoxy, $C_1$-$C_4$-Halogenalkoxy, $C_1$-$C_4$-Halogenalkyl, Halogen, Cyan oder Nitro, bedeuten

$R^1$, $R^2$ unabhängig voneinander Wasserstoff, $C_1$-$C_6$-Alkyl oder $R^1$ und $R^2$, zusammen eine Methylenkette mit 2 bis 6 Methylengruppen bedeuten,

Ar einen ein- oder zweikernigen Arylrest bedeutet, der ggf. ein bis dreifach substituiert ist durch $C_1$-$C_4$-Alkyl, $C_1$-$C_4$-Alkoxy, $C_1$-$C_4$-Halogenalkyl, Phenoxy, Halogenphenoxy, Benzyloxy, Phenyl, $C_1$-$C_4$-Halogenalkoxy,Hydroxy, Halogen oder Nitro und

n für 0 - 3 steht,

behandelt.

4. Verfahren zur Herstellung eines N-Fumaramidsäurederivate der Formel

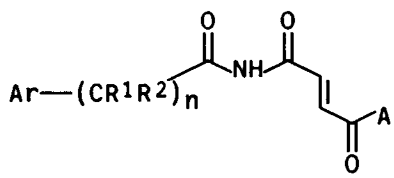

in welcher

A für O-$R^3$ oder $NR^4R^5$ steht, wobei

$R^3$, $R^4$, $R^5$ unabhängig voneinander Wasserstoff, $C_1$-$C_6$-Alkyl, $C_1$-$C_4$-Halogenalkyl, $C_2$-$C_6$-Alkenyl, $C_2$-$C_6$-Alkinyl, Aralkyl mit 1-4 Kohlenstoffatomen im Alkylteil und der Arylrest ggf. ein- bis dreifach substituiert ist durch Substituenten aus der Gruppe $C_1$-$C_4$-Alkyl, $C_1$-$C_4$-Alkoxy, $C_1$-$C_4$-Halogenalkoxy, $C_1$-$C_4$-Halogenalkyl, Halogen, Cyan oder Nitro, bedeuten

$R^1$, $R^2$ unabhängig voneinander Wasserstoff, $C_1$-$C_6$-Alkyl oder $R^1$ und $R^2$, zusammen eine Methylenkette mit 2 bis 6 Methylengruppen bedeuten,

Ar einen ein- oder zweikernigen Arylrest bedeutet, der ggf. ein bis dreifach substituiert ist durch $C_1$-$C_4$-Alkyl, $C_1$-$C_4$-Alkoxy, $C_1$-$C_4$-Halogenalkyl, Phenoxy, Halogenphenoxy, Benzyloxy, Phenyl, $C_1$-$C_4$-Halogenalkoxy,Hydroxy, Halogen oder Nitro und

n für 0 - 3 steht,

dadurch gekennzeichnet, daß man ein Fumarsäuremonochloridmonoester-Derivat der Formel II

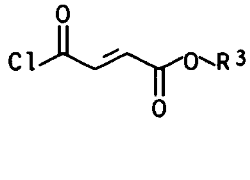

II

mit einem Imidoester III

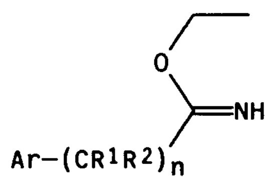

umsetzt oder Fumaramidsäure IV

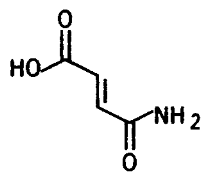

mit Chlorameisensäureethylester umsetzt und danach mit dem Imidoester III umsetzt.

5. Verbindung der Formel 1 gemäß Anspruch 1, dadurch gekennzeichnet, daß A $NH_2$, n 1, $R^1$ und $R^2$ Wasserstoff und Ar 4-Methoxyphenyl bedeutet.

6. Verbindung der Formel 1 gemäß Anspruch 1, dadurch gekennzeichnet, daß A Äthoxy, n 1, $R^1$ und $R^2$ Wasserstoff und Ar 4-Methylphenyl bedeutet.

7. Verbindung der Formel 1 gemäß Anspruch 1, dadurch gekennzeichnet, daß A Methoxy, n 1, $R^1$ und $R^2$ Wasserstoff und Ar Phenyl bedeutet.

Europäisches Patentamt

# EUROPÄISCHER RECHERCHENBERICHT

Nummer der Anmeldung

**EP 91 11 6817**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | CHEMICAL ABSTRACTS Band 62, Nr. 3, 1. Februar 1965, Seite 2770, Zusammenfassung Nr. 2770e, Columbus, Ohio, US; I.V. SMOLANKA et al.: "4(5)-Oxazolones and 4(5)-thiazolones. III. N-Acyl, N-thioacrylamides of cinnamic and maleic acids and their bromocyclization" & Ukr. Khim. Zh. 1964, Band 30, Nr. 9, Seiten 950-953<br>– – – | 1 | C 07 C 235/88<br>A 01 N 37/30 |
| X | CHEMICAL ABSTRACTS Band 85, Nr. 9, 30. August 1976, Seite 443, Zusammenfassung Nr. 61412n, Columbus, Ohio, US;<br>& JP - A - 76032789 (TAKEDA CHEMICAL INDUSTRIES LTD.) 19.03.1976 (Kat. D)<br>– – – | 1 | |
| X,D | THE JOURNAL OF ANTIBIOTICS Band 28, Nr. 9, September 1975, Seiten 648-655; Y. SUHARA et al.: "A new antibiotic, fumaramidmycin"<br>* Seiten 651,652 *<br>– – – | 1 | |
| X | CHEMICAL ABSTRACTS Band 84, Nr. 7, 16. Februar 1976, Seite 323, Zusammenfassung Nr. 41900w, Columbus, Ohio, US; T. HASEGAWA et al.: "New antibiotic, C-9154" & J. Antibiot. 1975, Band 28, Nr. 9, Seiten 713-716<br>– – – | 1 | **RECHERCHIERTE SACHGEBIETE (Int. Cl.5)** |
| A,D | THE JOURNAL OF ORGANIC CHEMISTRY Band 30, Nr. 3, März 1965, Seiten 699-702, American Chemical Society; R.H. DEWOLFE et al.: "Mechanism of Hydrolysis of Ethyl Benzimidates in Acidic Solutions"<br>* Seiten 699,700 *<br>– – – | 4 | C 07 C 235/00<br>A 01 N 37/00 |
| A,D | JOURNAL OF MEDICINAL CHEMISTRY Band 29, Nr. 10, 1986, Seiten 1868-1871; J.W. SCHOENECKER et al.: "Irreversible Blockage of Opioid Receptor Types by Ester Homologues of beta-Funaltrexamine"<br>* Zusammenfassung *<br>– – – – – | 4 | |

Der vorliegende Recherchenbericht wurde für alle Patentansprüche erstellt

| Recherchenort | Abschlußdatum der Recherche | Prüfer |
|---|---|---|
| Berlin | 03 Dezember 91 | RUFET J.M.A. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument